# EUROPEAN PATENT SPECIFICATION

(11) **EP 4 110 958 B1**
(45) Date of publication and mention of the grant of the patent: **10.09.2025**
(21) Application number: 22767559.2
(22) Date of filing: 11.03.2022
(51) Int. Cl.: C12Q 1/689

(54) **PRIMER SET FOR DETECTING FOOD POISONING BACTERIA BY USING NEXT-GENERATION SEQUENCING METHOD AND METHOD FOR DETECTING FOOD POISONING BACTERIA BY USING THE PRIMER SET**
PRIMER-SET ZUM NACHWEIS VON LEBENSMITTELVERGIFTENDEN BAKTERIEN UNTER VERWENDUNG EINES SEQUENZIERUNGSVERFAHRENS DER NÄCHSTEN GENERATION UND VERFAHREN ZUM NACHWEIS VON LEBENSMITTELVERGIFTENDEN BAKTERIEN UNTER VERWENDUNG DES PRIMER-SETS
ENSEMBLE D'AMORCES POUR DÉTECTER DES BACTÉRIES D'INTOXICATION ALIMENTAIRE À L'AIDE D'UN PROCÉDÉ DE SÉQUENÇAGE DE NOUVELLE GÉNÉRATION ET PROCÉDÉ DE DÉTECTION DE BACTÉRIES D'INTOXICATION ALIMENTAIRE À L'AIDE DE L'ENSEMBLE D'AMORCES

(30) Priority: 11.03.2021 KR 20210032317; 10.03.2022 KR 20220029865
(43) Date of publication of application: 04.01.2023
(73) Proprietor: Sanigen Co., Ltd., Anyang-si, Gyeongi-do 14059 (KR); Korea Food & Drug Administration, Cheongju-si 28159 (KR)
(72) Inventor: PARK, Jeongwoong, Yongin-si Gyeonggi-do 16990 (KR); CHOI, Jinho, Seoul 06683 (KR); HA, Eunsu, Seoul 08788 (KR); KANG, Byungcheol, Gunpo-si Gyeonggi-do 15864 (KR); CHOI, Iseul, Anyang-si Gyeonggi-do 14033 (KR); LEE, Joseph, Seoul 08764 (KR); NA, Hongjun, Suwon-si Gyeonggi-do 16298 (KR); MOON, Hyejin, Uiwang-si Gyeonggi-do 16014 (KR); KIM, Soon Han, Cheongju-si Chungcheongbuk-do 28159 (KR); KIM, Seung Hwan, Cheongju-si Chungcheongbuk-do 28159 (KR); LEE, Woo Jung, Cheongju-si Chungcheongbuk-do 28159 (KR)
(74) Representative: Murgitroyd & Company
(86) International application number: PCT/KR2022/003456
(87) International publication number: WO 2022/191663

(56) References cited:
- JP-A- 2005 034 121
- JP-A- 2015 146 786
- KR-A- 20130 065 337
- US-A1- 2009 253 183
- FERRARIO CHIARA ET AL: "Next generation sequencing-based multigene panel for high throughput detection of food-borne pathogens", INTERNATIONAL JOURNAL OF FOOD MICROBIOLOGY, vol. 256, 3 May 2017 (2017-05-03), NL, pages 20 - 29, XP055965177, ISSN: 0168-1605, DOI: 10.1016/j.ijfoodmicro.2017.05.001
- HWANG JINIK ET AL: "Detection of coat protein gene of nervous necrosis virus using loop-mediated isothermal amplification", ASIAN PACIFIC JOURNAL OF TROPICAL MEDICINE, HAINAN MEDICAL COLLEGE, SINGAPORE, vol. 9, no. 3, 13 January 2016 (2016-01-13), pages 235 - 240, XP029461428, ISSN: 1995-7645, DOI: 10.1016/J.APJTM.2016.01.035
- LEE DOKYUNG ET AL: "Clinical Evaluation of a Loop-Mediated Isothermal Amplification (LAMP) Assay for Rapid Detection of Neisseria meningitidis in Cerebrospinal Fluid", 8 April 2015 (2015-04-08), XP093197486, Retrieved from the Internet <URL:https://journals.plos.org/plosone/article?id=10.1371/journal.pone.0122922> DOI: 10.1371/journal.pone.0122922
- FERRARIO CHIARA, LUGLI GABRIELE ANDREA, OSSIPRANDI MARIA CRISTINA, TURRONI FRANCESCA, MILANI CHRISTIAN, DURANTI SABRINA, MANCABELL: "Next generation sequencing-based multigene panel for high throughput detection of food-borne pathogens", INTERNATIONAL JOURNAL OF FOOD MICROBIOLOGY, vol. 256, 1 September 2017 (2017-09-01), NL , pages 20 - 29, XP055965177, ISSN: 0168-1605, DOI: 10.1016/j.ijfoodmicro.2017.05.001
- JAGADEESAN BALAMURUGAN; GERNER-SMIDT PETER; ALLARD MARC W; LEUILLET SÉBASTIEN; WINKLER ANETT; XIAO YINGHUA; CHAFFRON SAMUEL; VAN D: "The use of next generation sequencing for improving food safety: Translation into practice", FOOD MICROBIOLOGY, vol. 79, 1 June 2019 (2019-06-01), GB , pages 96 - 115, XP085574633, ISSN: 0740-0020, DOI: 10.1016/j.fm.2018.11.005
- KWON JOON-GI , KIM SEON-KYUN , LEE JU-HOON: "Recent next-generation sequencing and bioinformatic analysis methods for food microbiome research", FOOD SCIENCE AND INDUSTRY, vol. 52, no. 3, 30 September 2019 (2019-09-30), pages 220 - 228, XP055965190, ISSN: 0257-2397, DOI: 10.23093/FSI.2019.52.3.220

## Description

### Technical Field

The present invention relates to a primer set for detecting food poisoning bacteria by using a next-generation sequencing method and a method for detecting food poisoning bacteria by using the primer set.

### Background Art

Food poisoning refers to diseases associated with infection or toxin caused by consumption of food or water, and it can cause symptoms such as vomiting, diarrhea, abdominal pain, and fever, and can cause, in severe cases, death. As the number of food poisoning outbreaks continues to increase with the development of group meals, food industry, and ready-to-eat food continue to increase, and as the import and export of food increases internationally, it is necessary to quickly and economically identify food poisoning in order to meet consumer demands for food safety or meet international distribution regulations.

Food poisoning is caused by bacteria, viruses, chemicals, parasites, and so on, and food can be contaminated in various ways throughout the entire flow from food production to cultivation, packaging, distribution, and consumption. Food poisoning bacteria that cause food poisoning are generally identified by biochemical or immunological methods after incubating a sample in a selective medium and isolating the bacteria. An immunological method can detect bacteria with high accuracy, but it costs a lot because it requires a large amount of sample and requires different antibodies for different bacteria. In addition, it is difficult to store and use antibodies, only one type of or a limited number of bacteria can be detected at a time by using an antibody, and detection time is long.

In recent years, polymerase chain reaction (PCR) has begun to be used for detecting food poisoning bacteria, and the demand is increasing due to its high accuracy, simplicity and quickness. In particular, a real-time PCR method has been obtaining high popularity because it has advantageous, for example, in that electrophoresis does not need to be performed since a PCR amplification product can be detected in real time, and in that not only detection accuracy and sensitivity are excellent but also detection reproducibility is high, detection can be automated, and a quantified detection result can be obtained. In addition, as a probe labeled with a fluorescent marker is used, less amount of a sample is needed than when detection is performed by using a DNA chip or an antigen-antibody reaction method.

Korean Patent No. 10-1456646, which is directed to a kit for detecting food poisoning bacteria and a method for detecting food poisoning bacteria by using the kit, discloses a kit for detecting food poisoning bacteria that can efficiently detect various types of food poisoning bacteria at the same time.

Further primer sets for detecting food poisoning bacteria and methods using these are disclosed by Ferrario, C. et al. Int J Food Microbiol (2017) 256:20-29 and JP 2015 146786.

### Disclosure of Invention

### Technical Problem

An object of the present invention is to provide a primer set for detecting food poisoning bacteria.

Another object of the present invention is to provide a composition comprising the primer set and a method of using the primer set.

### Solution to Problem

In order to achieve the above objects, the present invention provides a primer set as defined in the appendant claims.

In addition, the present invention provides a composition for detecting food poisoning bacteria, the composition comprising the primer set.

In addition, the present invention provides a next-generation sequencing panel for detecting food poisoning bacteria, the panel comprising the primer set.

In addition, the present invention provides a kit for detecting food poisoning bacteria, the kit comprising the primer set.

In addition, the present invention provides a next-generation sequencing method, comprising the steps of: amplifying a target sequence by using the primer set and a sample; preparing a library by using the amplified target sequence; and performing a next-generation sequencing by using the prepared library.

In addition, the present invention provides a method for detecting food poisoning bacteria, the method comprising the step of amplifying by using the primer set and a sample.

### Advantageous Effects of Invention

With the primer set according to the present invention, 16 types of food poisoning bacteria can be detected at one time, for a shorter time, and with high accuracy, and the primer set can be useful for simultaneous detection of food poisoning bacteria.

### Brief Description of Drawings

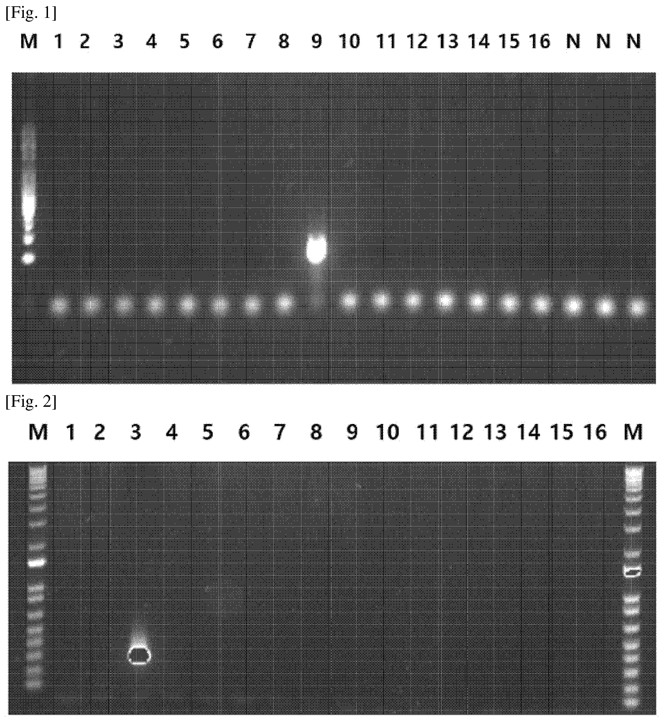

Figure 1 is an agarose gel electrophoresis analysis photograph that shows the result of detecting *Bacillus cereus* by using the 1^{st} primer pair according to the present invention (M: DNA marker, *1: Campylobacter jejuni, 2: Campylobacter coli, 3: Clostridium perfringens, 4: Vibrio parahaemolyticus, 5: Vibrio vulnificus, 6: Vibrio cholerae, 7: Salmonella typhimurium, 8: Staphylococcus aureus, 9: Bacillus cereus, 10: Yersinia enterocolitica,* 11: *Listeria monocytogenes,* 12: EHEC, 13: ETEC, 14: EPEC, 15: EIEC, 16: EAEC, and N: no template).
Figure 2 is an agarose gel electrophoresis analysis photograph that shows the result of detecting *Bacillus cereus* by using the 2^{nd} primer pair according to the present invention (M: DNA marker, *1: Staphylococcus aureus, 2: Yersinia enterocolitica, 3: Bacillus cereus, 4: Listeria monocytogenes, 5: Clostridium perfringens,* 6: EHEC, 7: EIEC, 8: EAEC, 9: ETEC, 10: EPEC, 11: *Salmonella typhimurium, 12: Vibrio vulnificus,* 13: *Vibrio cholerae,* 14: *Vibrio parahaemolyticus,* 15: *Campylobacter jejuni* , and 16: *Campylobacter coli).*

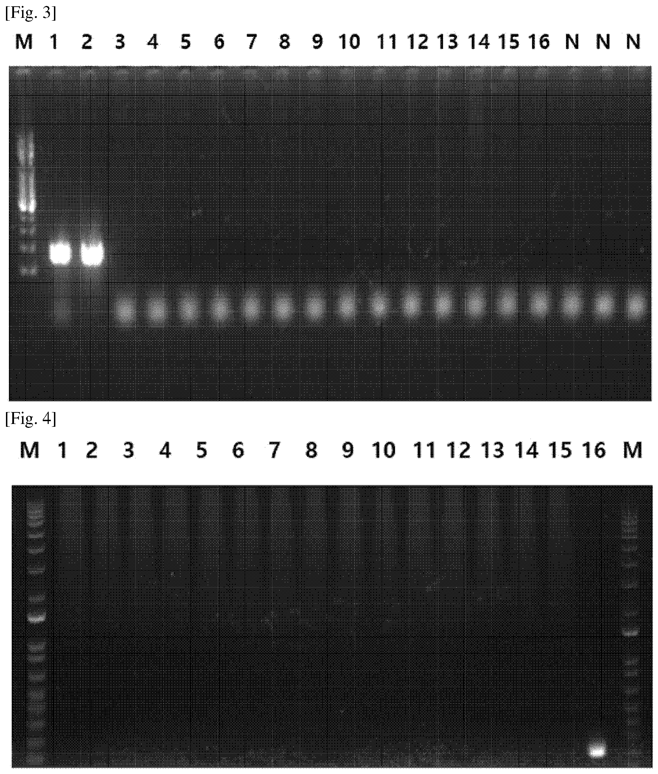

Figure 3 is an agarose gel electrophoresis photograph that shows the result of detecting *Campylobacter* by using the 3^{rd} primer pair according to the present invention (M: DNA marker, *1: Campylobacter jejuni, 2: Campylobacter coli, 3: Clostridium perfringens, 4: Vibrio parahaemolyticus, 5: Vibrio vulnificus, 6: Vibrio cholerae, 7: Salmonella typhimurium, 8: Staphylococcus aureus, 9: Bacillus cereus, 10: Yersinia enterocolitica,* 11: *Listeria monocytogenes,* 12: EHEC, 13: ETEC, 14: EPEC, 15: EIEC, 16: EAEC, and N: no template).
Figure 4 is an agarose gel electrophoresis photograph that shows the result of detecting *Campylobacter coli* by using the 4^{th} primer pair according to the present invention (M: DNA marker, *1: Staphylococcus aureus, 2: Yersinia enterocolitica, 3: Bacillus cereus, 4: Listeria monocytogenes, 5: Clostridium perfringens,* 6: EHEC, 7: EIEC, 8: EAEC, 9: ETEC, 10: EPEC, 11: *Salmonella typhimurium, 12: Vibrio vulnificus,* 13: *Vibrio cholerae,* 14: *Vibrio parahaemolyticus,* 15: *Campylobacter jejuni* , and 16: *Campylobacter coli).*

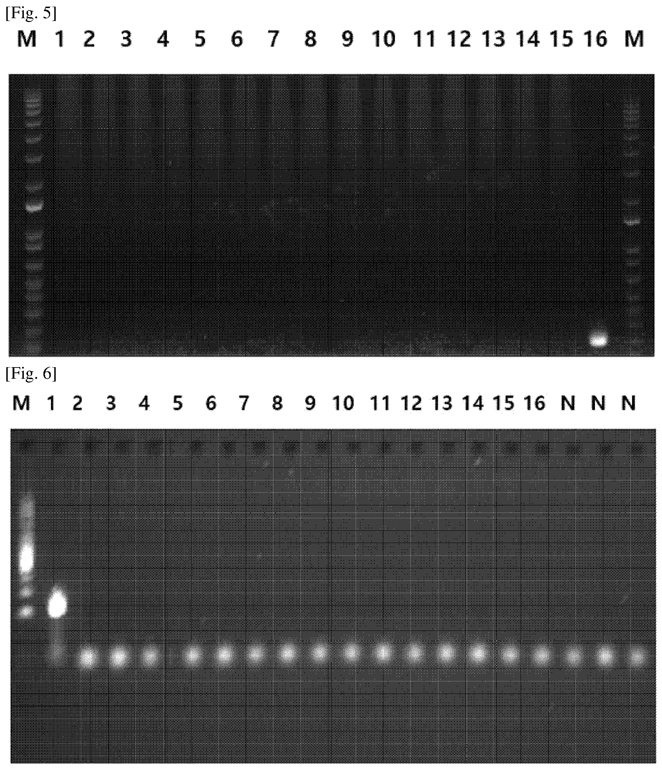

Figure 5 is an agarose gel electrophoresis analysis photograph that shows the result of detecting *Campylobacter coli* by using the 5^{th} primer pair according to the present invention (M: DNA marker, *1: Staphylococcus aureus, 2: Yersinia enterocolitica, 3: Bacillus cereus, 4: Listeria monocytogenes, 5: Clostridium perfringens,* 6: EHEC, 7: EIEC, 8: EAEC, 9: ETEC, 10: EPEC, 11: *Salmonella typhimurium, 12: Vibrio vulnificus,* 13: *Vibrio cholerae,* 14: *Vibrio parahaemolyticus,* 15: *Campylobacter jejuni* , and 16: *Campylobacter coli).*
Figure 6 is an agarose gel electrophoresis photograph that shows the result of detecting *Campylobacter jejuni* by using the 6^{th} primer pair according to the present invention (M: DNA marker, *1: Campylobacter jejuni, 2: Campylobacter coli, 3: Clostridium perfringens, 4: Vibrio parahaemolyticus, 5: Vibrio vulnificus, 6: Vibrio cholerae, 7: Salmonella typhimurium, 8: Staphylococcus aureus, 9: Bacillus cereus, 10: Yersinia enterocolitica,* 11: *Listeria monocytogenes,* 12: EHEC, 13: ETEC, 14: EPEC, 15: EIEC, 16: EAEC, and N: no template).

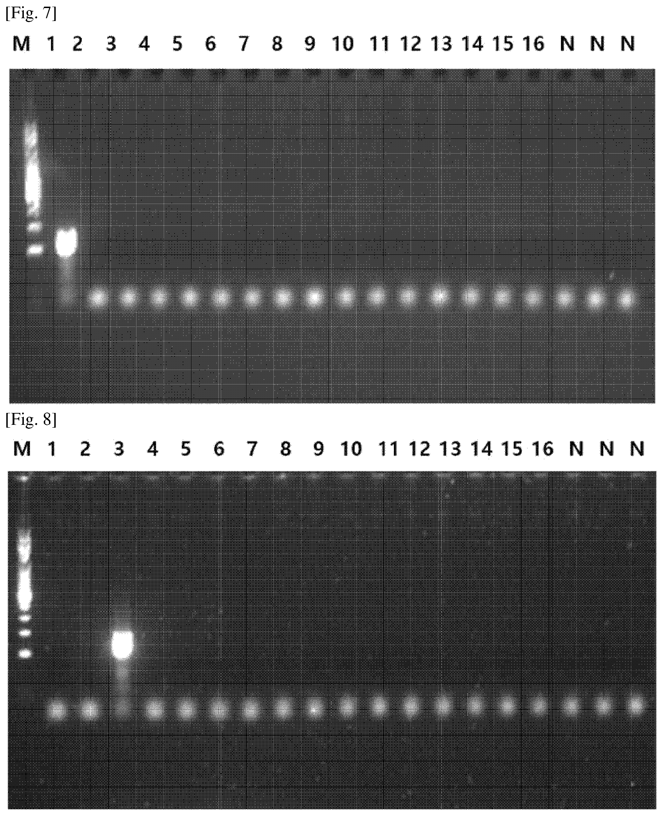

Figure 7 is an agarose gel electrophoresis photograph that shows the result of detecting *Campylobacter jejuni* by using the 7^{th} primer pair according to the present invention (M: DNA marker, *1: Campylobacter jejuni, 2: Campylobacter coli, 3: Clostridium perfringens, 4: Vibrio cholerae, 5: Vibrio parahaemolyticus, 6: Vibrio vulnificus, 7: Salmonella typhimurium, 8: Listeria monocytogenes, 9: Bacillus cereus, 10: Yersinia enterocolitica,* 11: *Staphylococcus aureus,* 12: EHEC, 13: ETEC, 14: EPEC, 15: EIEC, 16: EAEC, and N: no template).
Figure 8 is an agarose gel electrophoresis photograph that shows the result of detecting *Clostridium perfringens* by using the 8^{th} primer pair according to the present invention (M: DNA marker, *1: Campylobacter jejuni, 2: Campylobacter coli, 3: Clostridium perfringens, 4: Vibrio cholerae, 5: Vibrio parahaemolyticus, 6: Vibrio vulnificus, 7: Salmonella typhimurium, 8: Listeria monocytogenes, 9: Bacillus cereus, 10: Yersinia enterocolitica,* 11: *Staphylococcus aureus,* 12: EHEC, 13: ETEC, 14: EPEC, 15: EIEC, 16: EAEC, and N: no template).

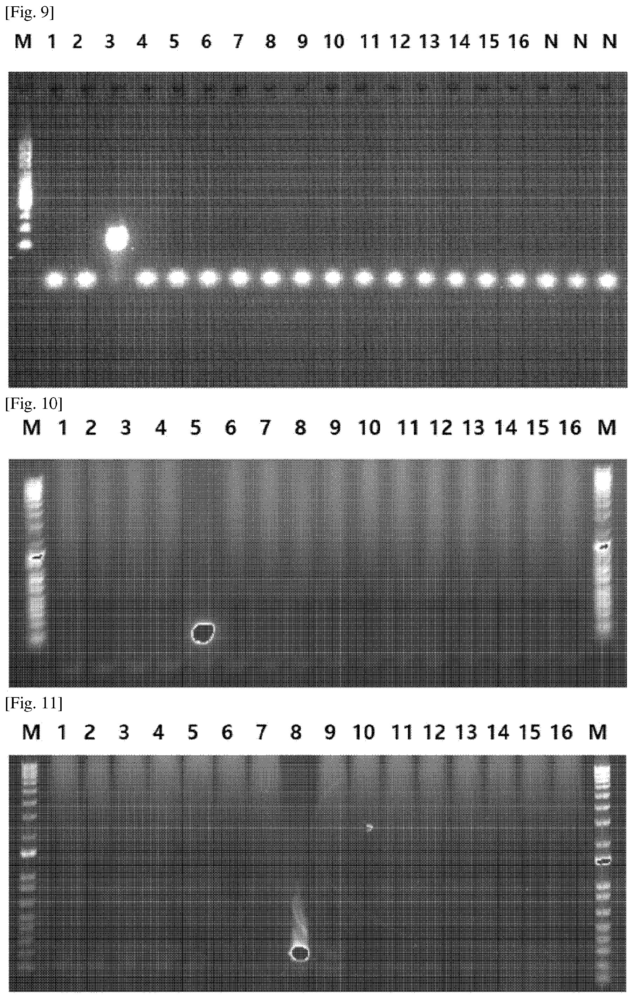

Figure 9 is an agarose gel electrophoresis analysis photograph that shows the result of detecting *Clostridium perfringens* using the 9^{th} primer pair according to the present invention (M: DNA marker, *1: Campylobacter jejuni, 2: Campylobacter coli, 3: Clostridium perfringens, 4: Vibrio parahaemolyticus, 5: Vibrio vulnificus, 6: Vibrio cholerae, 7: Salmonella typhimurium, 8: Staphylococcus aureus, 9: Bacillus cereus, 10: Yersinia enterocolitica,* 11: *Listeria monocytogenes,* 12: EHEC, 13: ETEC, 14: EPEC, 15: EIEC, 16: EAEC, and N: no template).
Figure 10 is an agarose gel electrophoresis photograph that shows the result of detecting *Clostridium perfringens* by using the 10^{th} primer pair according to the present invention (M: DNA marker, *1: Staphylococcus aureus, 2: Yersinia enterocolitica, 3: Bacillus cereus, 4: Listeria monocytogenes, 5: Clostridium perfringens,* 6: EHEC, 7: EIEC, 8: EAEC, 9: ETEC, 10: EPEC, 11: *Salmonella typhimurium, 12: Vibrio vulnificus,* 13: *Vibrio cholerae,* 14: *Vibrio parahaemolyticus,* 15: *Campylobacter jejuni* , 16: *Campylobacter coli).*
Figure 11 is an agarose gel electrophoresis analysis photograph that shows the result of detecting EAEC by using the 11^{th} primer pair according to the present invention (M: DNA marker, *1: Staphylococcus aureus, 2: Yersinia enterocolitica, 3: Bacillus cereus, 4: Listeria monocytogenes, 5: Clostridium perfringens,* 6: EHEC, 7: EIEC, 8: EAEC, 9: ETEC, 10: EPEC, 11: *Salmonella typhimurium, 12: Vibrio vulnificus,* 13: *Vibrio cholerae,* 14: *Vibrio parahaemolyticus,* 15: *Campylobacter jejuni,* and 16: *Campylobacter coli).*

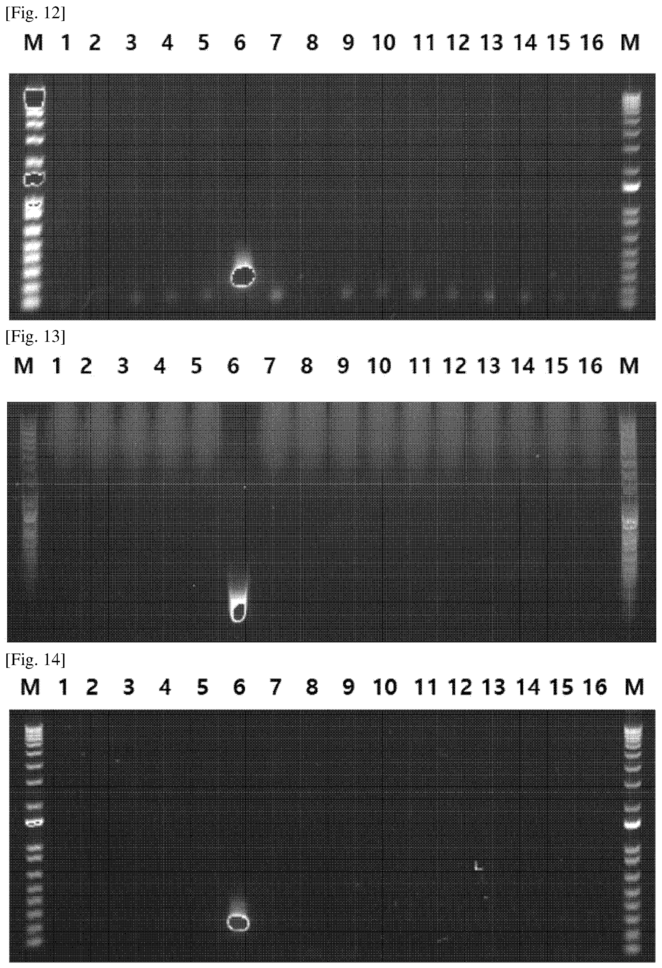

Figure 12 is an agarose gel electrophoresis analysis photograph that shows the result of detecting EHEC by using the 12^{th} primer pair according to the present invention (M: DNA marker, *1: Staphylococcus aureus, 2: Yersinia enterocolitica, 3: Bacillus cereus, 4: Listeria monocytogenes, 5: Clostridium perfringens,* 6: EHEC, 7: EIEC, 8: EAEC, 9: ETEC, 10: EPEC, 11: *Salmonella typhimurium, 12: Vibrio vulnificus,* 13: *Vibrio cholerae,* 14: *Vibrio parahaemolyticus,* 15: *Campylobacter jejuni,* and 16: *Campylobacter coli).*
Figure 13 is an Agarose gel electrophoresis analysis photograph that shows the result of detecting EHEC by using the 13^{th} primer pair according to the present invention (M: DNA marker, *1: Staphylococcus aureus, 2: Yersinia enterocolitica, 3: Bacillus cereus, 4: Listeria monocytogenes, 5: Clostridium perfringens,* 6: EHEC, 7: EIEC, 8: EAEC, 9: ETEC, 10: EPEC, 11: *Salmonella typhimurium, 12: Vibrio vulnificus,* 13: *Vibrio cholerae,* 14: *Vibrio parahaemolyticus,* 15: *Campylobacter jejuni,* and 16: *Campylobacter coli).*
Figure 14 is an agarose gel electrophoresis analysis photograph that shows the result of detecting EHEC by using the 14^{th} primer pair according to the present invention (M: DNA marker, *1: Staphylococcus aureus, 2: Yersinia enterocolitica, 3: Bacillus cereus, 4: Listeria monocytogenes, 5: Clostridium perfringens,* 6: EHEC, 7: EIEC, 8: EAEC, 9: ETEC, 10: EPEC, 11: *Salmonella typhimurium, 12: Vibrio vulnificus,* 13: *Vibrio cholerae,* 14: *Vibrio parahaemolyticus,* 15: *Campylobacter jejuni,* and 16: *Campylobacter coli).*

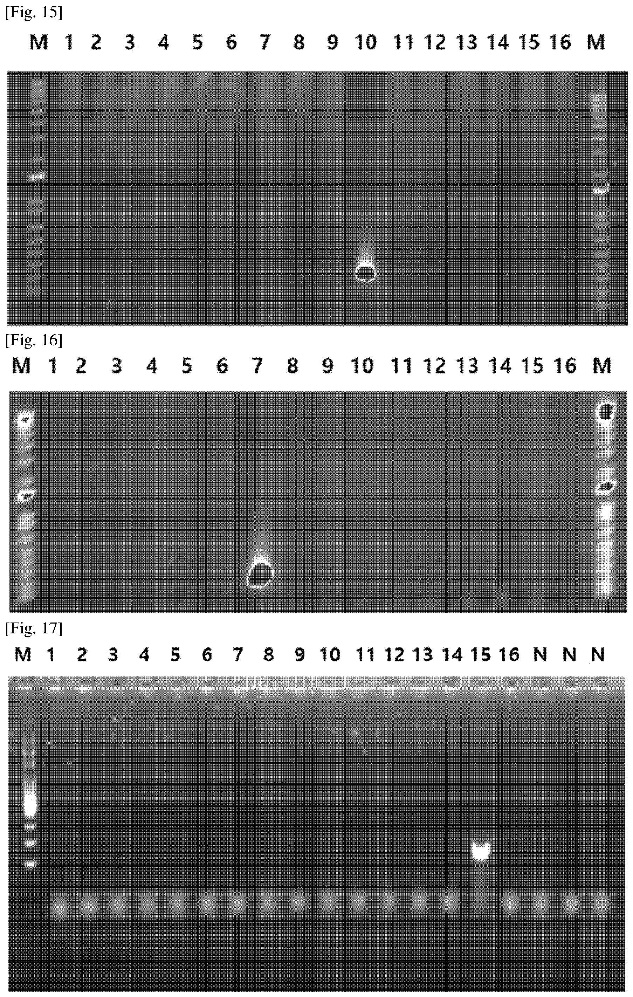

Figure 15 is an agarose gel electrophoresis analysis photograph that shows the result of detecting EPEC by using the 15^{th} primer pair according to the present invention (M: DNA marker, *1: Staphylococcus aureus, 2: Yersinia enterocolitica, 3: Bacillus cereus, 4: Listeria monocytogenes, 5: Clostridium perfringens,* 6: EHEC, 7: EIEC, 8: EAEC, 9: ETEC, 10: EPEC, 11: *Salmonella typhimurium, 12: Vibrio vulnificus,* 13: *Vibrio cholerae,* 14: *Vibrio parahaemolyticus,* 15: *Campylobacter jejuni,* and 16: *Campylobacter coli).*
Figure 16 is an agarose gel electrophoresis analysis photograph that shows the result of detecting EIEC by using the 16^{th} primer pair according to the present invention (M: DNA marker, *1: Staphylococcus aureus, 2: Yersinia enterocolitica, 3: Bacillus cereus, 4: Listeria monocytogenes, 5: Clostridium perfringens,* 6: EHEC, 7: EIEC, 8: EAEC, 9: ETEC, 10: EPEC, 11: *Salmonella typhimurium, 12: Vibrio vulnificus,* 13: *Vibrio cholerae,* 14: *Vibrio parahaemolyticus,* 15: *Campylobacter jejuni,* and 16: *Campylobacter coli).*
Figure 17 is an agarose gel electrophoresis analysis photograph that shows the result of detecting EIEC by using the 17^{th} primer pair according to the present invention (M: DNA marker, *1: Campylobacter jejuni, 2: Campylobacter coli, 3: Clostridium perfringens, 4: Vibrio cholerae, 5: Vibrio parahaemolyticus, 6: Vibrio vulnificus, 7: Salmonella typhimurium, 8: Listeria monocytogenes, 9: Bacillus cereus,* 10: *Yeshonia Enterocolitica,* 11: *Staphylococcus aureus,* 12: EHEC, 13: ETEC, 14: EPEC, 15: EIEC, 16: EAEC, and N: no template).

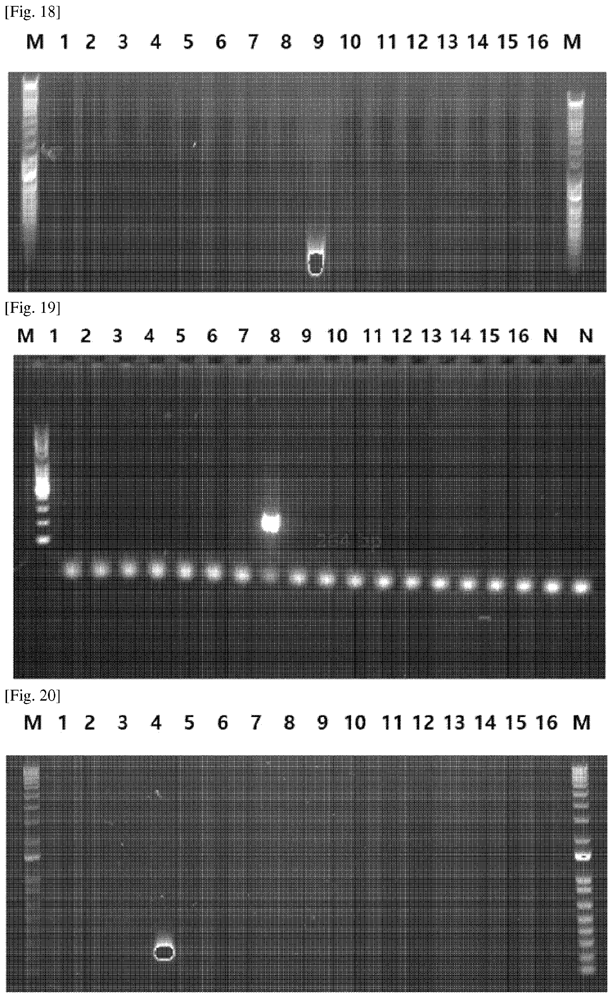

Figure 18 is an agarose gel electrophoresis analysis photograph that shows the result of detecting ETEC by using the 18^{th} primer pair according to the present invention (M: DNA marker, *1: Staphylococcus aureus, 2: Yersinia enterocolitica, 3: Bacillus cereus, 4: Listeria monocytogenes, 5: Clostridium perfringens,* 6: EHEC, 7: EIEC, 8: EAEC, 9: ETEC, 10: EPEC, 11: *Salmonella typhimurium, 12: Vibrio vulnificus,* 13: *Vibrio cholerae,* 14: *Vibrio parahaemolyticus,* 15: *Campylobacter jejuni,* and 16: *Campylobacter coli).*
Figure 19 is an agarose gel electrophoresis photograph that shows the result of detecting *Listeria monocytogenes* by using the 19^{th} primer pair according to the present invention (M: DNA marker, *1: Campylobacter jejuni, 2: Campylobacter coli, 3: Clostridium perfringens, 4: Vibrio cholerae, 5: Vibrio parahaemolyticus, 6: Vibrio vulnificus, 7: Salmonella typhimurium, 8: Listeria monocytogenes, 9: Bacillus cereus, 10: Yersinia enterocolitica,* 11: *Staphylococcus aureus,* 12: EHEC, 13: ETEC, 14: EPEC, 15: EIEC, 16: EAEC, and N: no template).
Figure 20 is an agarose gel electrophoresis photograph that shows the result of detecting *Listeria monocytogenes* by using the 20^{th} primer pair according to the present invention (M: DNA marker, *1: Staphylococcus aureus, 2: Yersinia enterocolitica, 3: Bacillus cereus, 4: Listeria monocytogenes, 5: Clostridium perfringens,* 6: EHEC, 7: EIEC, 8: EAEC, 9: ETEC, 10: EPEC, 11: *Salmonella typhimurium, 12: Vibrio vulnificus,* 13: *Vibrio cholerae,* 14: *Vibrio parahaemolyticus,* 15: *Campylobacter jejuni* , and 16: *Campylobacter coli).*

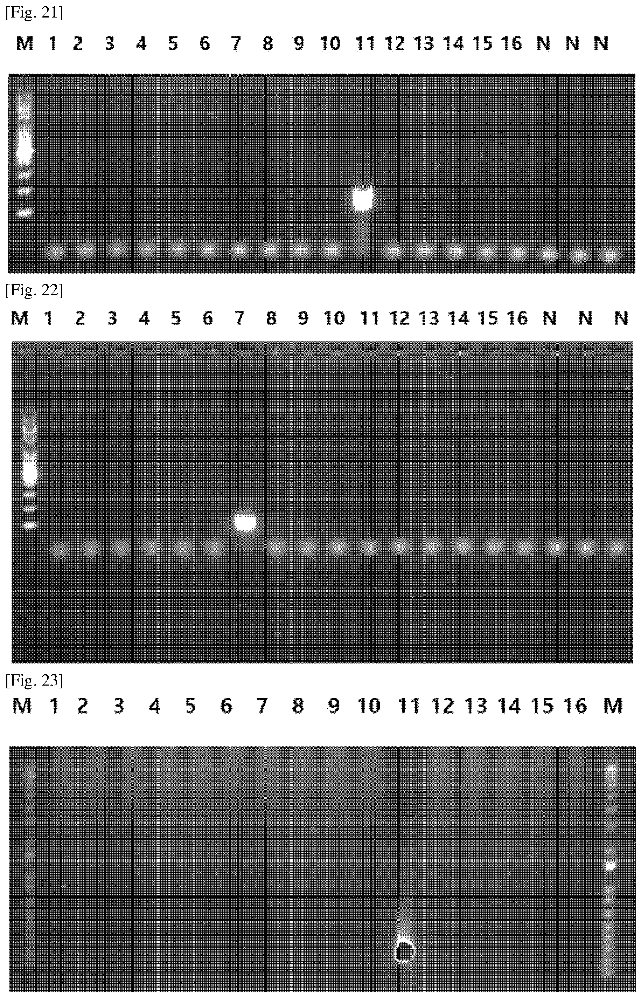

Figure 21 is an agarose gel electrophoresis photograph that shows the result of detecting *Listeria monocytogenes* by using the 21^{st} primer pair according to the present invention (M: DNA marker, *1: Campylobacter jejuni, 2: Campylobacter coli, 3: Clostridium perfringens, 4: Vibrio parahaemolyticus, 5: Vibrio vulnificus, 6: Vibrio cholerae, 7: Salmonella typhimurium, 8: Staphylococcus aureus, 9: Bacillus cereus, 10: Yersinia enterocolitica,* 11: *Listeria monocytogenes,* 7: 12: EHEC, 13: ETEC, 14: EPEC, 15: EIEC, 16: EAEC, and N: no template).
Figure 22 is an agarose gel electrophoresis analysis photograph that shows the result of detecting *Salmonella typhimurium* by using the 22^{nd} primer pair according to the present invention (M: DNA marker, *1: Campylobacter jejuni, 2: Campylobacter coli, 3: Clostridium perfringens, 4: Vibrio parahaemolyticus, 5: Vibrio vulnificus, 6: Vibrio cholerae, 7: Salmonella typhimurium, 8: Staphylococcus aureus, 9: Bacillus cereus, 10: Yersinia enterocolitica,* 11: *Listeria monocytogenes,* 12: EHEC, 13: ETEC, 14: EPEC, 15: EIEC, 16: EAEC, and N: no template).
Figure 23 is an agarose gel electrophoresis analysis photograph that shows the result of detecting *Salmonella typhimurium* by using the 23^{rd} primer pair according to the present invention (M: DNA marker, *1: Staphylococcus aureus, 2: Yersinia enterocolitica, 3: Bacillus cereus, 4: Listeria monocytogenes, 5: Clostridium perfringens, 6:* EHEC, 7: EIEC, 8: EAEC, 9: ETEC, 10: EPEC, 11: *Salmonella typhimurium, 12: Vibrio vulnificus,* 13: *Vibrio cholerae,* 14: *Vibrio parahaemolyticus,* 15: *Campylobacter jejuni,* and 16: *Campylobacter coli).*

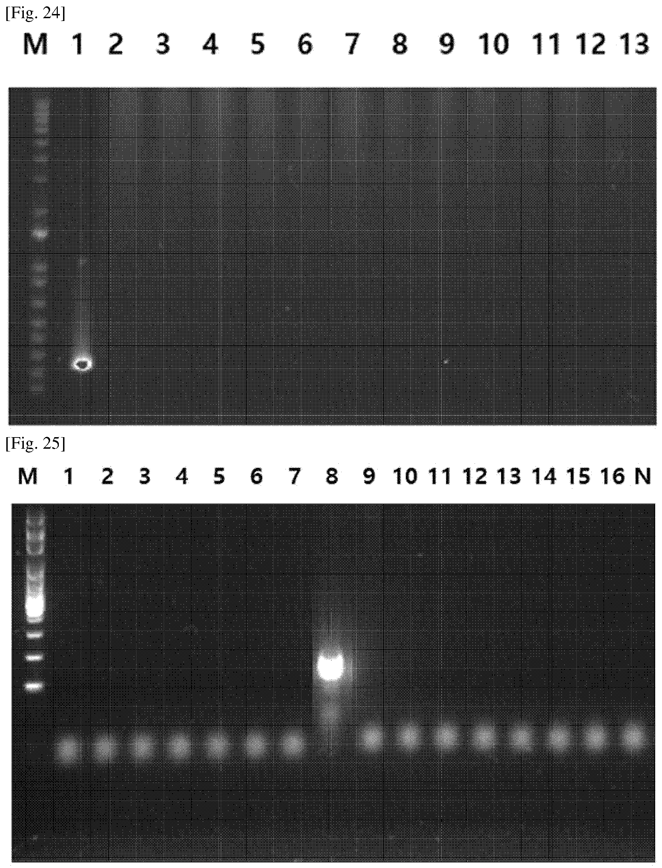

Figure 24 is an agarose gel electrophoresis analysis photograph that shows the result of detecting *Staphylococcus aureus* by using the 24^{th} primer pair according to the present invention (M: DNA marker, *1: Staphylococcus aureus, 2: Yersinia enterocolitica, 3: Bacillus cereus, 4: Listeria monocytogenes, 5: Clostridium perfringens, 6:* EHEC, 7: EIEC, 8: *Salmonella typhimurium, 9: Vibrio vulnificus,* 10: *Vibrio cholerae,* 11: *Vibrio parahaemolyticus, 12: Campylobacter jejuni,* and 13: *Campylobacter coli).*
Figure 25 is an agarose gel electrophoresis photograph that shows the result of detecting *Staphylococcus aureus* by using the 25^{th} primer pair according to the present invention (M: DNA marker, *1: Campylobacter jejuni, 2: Campylobacter coli, 3: Clostridium perfringens, 4: Vibrio cholerae, 5: Vibrio parahaemolyticus, 6: Vibrio vulnificus, 7: Salmonella typhimurium, 8: Staphylococcus aureus, 9: Listeria monocytogenes,* 10: *Bacillus cereus,* 11: *Yersinia enterocolitica,* 12: EHEC, 13: ETEC, 14: EPEC, 15: EIEC, 16: EAEC, and N: no template).

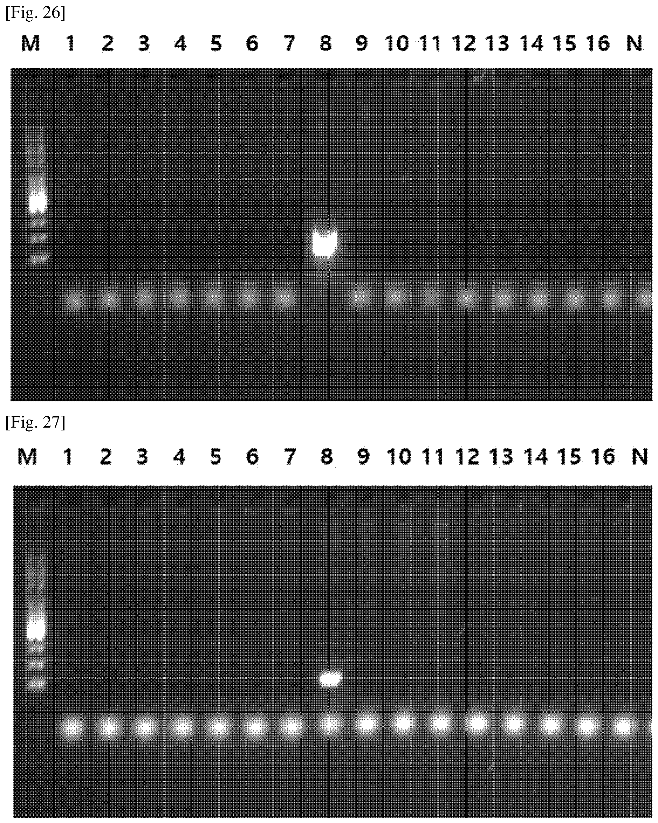

Figure 26 is an agarose gel electrophoresis photograph that shows the result of detecting *Staphylococcus aureus* by using the 26^{th} primer pair according to the present invention (M: DNA marker, *1: Campylobacter jejuni, 2: Campylobactercoli, 3: Clostridium perfringens, 4: Vibrio cholerae, 5: Vibrio parahaemolyticus, 6: Vibrio vulnificus, 7: Salmonella typhimurium, 8: Staphylococcus aureus, 9: Listeria monocytogenes,* 10: *Bacillus cereus,* 11: *Yesinia enterocolitica,* 12: EHEC, 13: ETEC, 14: EPEC, 15: EIEC, 16: EAEC, and N: no template).
Figure 27 is an agarose gel electrophoresis photograph that shows the result of detecting *Staphylococcus aureus* by using the 27^{th} primer pair according to the present invention (M: DNA marker, *Campylobacter jejuni, 2: Campylobacter coli, 3: Clostridium perfringens, 4: Vibrio cholerae, 5: Vibrio parahaemolyticus, 6: Vibrio vulnificus, 7: Salmonella typhimurium, 8: Staphylococcus aureus, 9: Listeria monocytogenes,* 10: *Bacillus cereus,* 11: *Yersinia enterocolitica,* 12: EHEC, 13: ETEC, 14: EPEC, 15: EIEC, 16: EAEC, and N: no template).

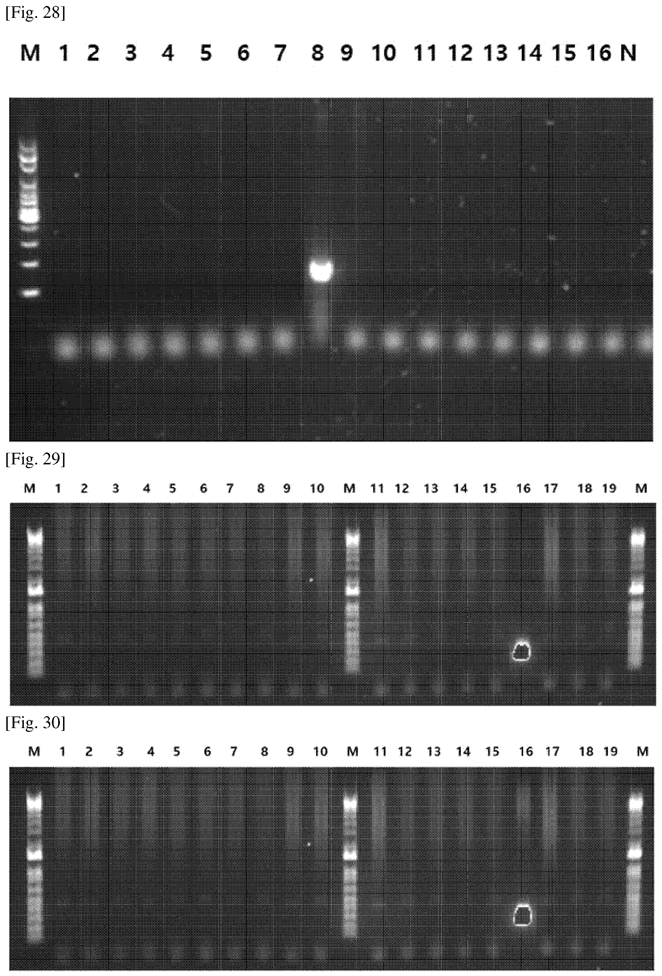

Figure 28 is an agarose gel electrophoresis analysis photograph that shows the result of detecting *Staphylococcus aureus* by using the 28^{th} primer pair according to the present invention (M: DNA marker, *1: Campylobacter jejuni, 2: Campylobacter coli, 3: Clostridium perfringens, 4: Vibrio cholerae, 5: Vibrio parahaemolyticus, 6: Vibrio vulnificus, 7: Salmonella typhimurium, 8: Staphylococcus aureus, 9: Listeria monocytogenes,* 10: *Bacillus cereus,* 11: *Yersinia enterocolitica,* 12: EHEC, 13: ETEC, 14: EPEC, 15: EIEC, 16: EAEC, and N: no template).
Figure 29 is an agarose gel electrophoresis photograph that shows the result of detecting *Vibrio cholerae* by using the 29^{th} primer pair according to the present invention (M: DNA marker, *1: Staphylococcus aureus* (ATCC25923), 2: *Staphylococcus aureus* (ATCC23235), 3: *Staphylococcus aureus* (CCARM), 4: *Staphylococcus aureus* (NewMan), 5: *Yersinia enterocolitica, 6: Bacillus cereus, 7: Listeria monocytogenes, 8: Clostridium perfringens,* 9: EHEC, 10: EIEC, 11: EAEC, 12: ETEC, 13: EPEC, 14: *Salmonella typhimurium,* 15: *Vibrio vulnificus,* 16: *Vibrio cholerae, 17: Vibrio parahaemolyticus,* 18: *Campylobacter jejuni,* and 19: *Campylobacter coli).*
Figure 30 is an agarose gel electrophoresis photograph that shows the result of detecting *Vibrio cholerae* by using the 30^{th} primer pair according to the present invention (M: DNA marker, *1: Staphylococcus aureus* (ATCC25923), 2: *Staphylococcus aureus* (ATCC23235), 3: *Staphylococcus aureus* (CCARM), 4: *Staphylococcus aureus* (NewMan), 5: *Yersinia enterocolitica, 6: Bacillus cereus, 7: Listeria monocytogenes, 8: Clostridium perfringens,* 9: EHEC, 10: EIEC, 11: EAEC, 12: ETEC, 13: EPEC, 14: *Salmonella typhimurium,* 15: *Vibrio vulnificus,* 16: *Vibrio cholerae, 17: Vibrio parahaemolyticus,* 18: *Campylobacter jejuni,* and 19: *Campylobacter coli*).

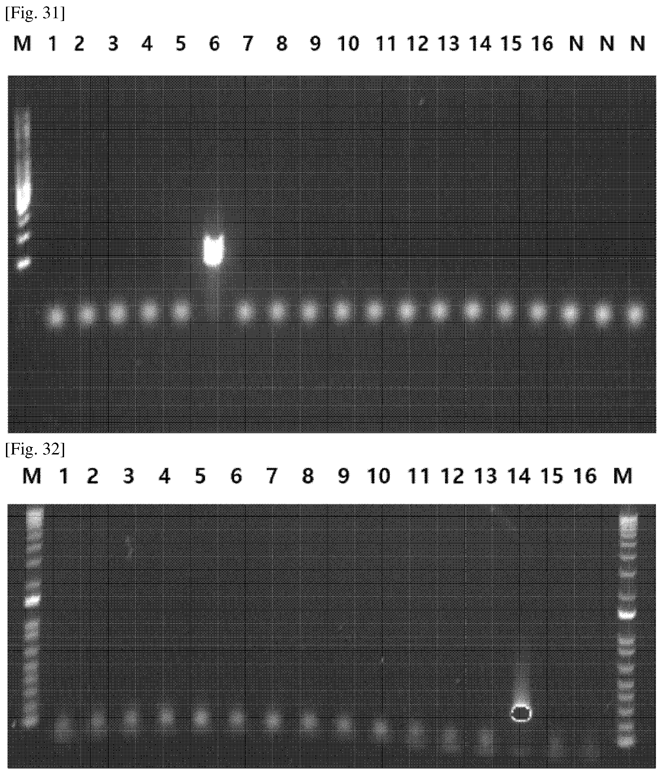

Figure 31 is an agarose gel electrophoresis photograph that shows the result of detecting *Vibrio cholerae* by using the 31^{st} primer pair according to the present invention (M: DNA marker, *1: Campylobacter jejuni, 2: Campylobacter coli, 3: Clostridium perfringens, 4: Vibrio parahaemolyticus, 5: Vibrio vulnificus, 6: Vibrio cholerae, 7: Salmonella typhimurium, 8: Staphylococcus aureus, 9: Bacillus cereus, 10: Yersinia enterocolitica,* 11: *Listeria monocytogenes,* 12: EHEC, 13: ETEC, 14: EPEC, 15: EIEC, 16: EAEC, and N: no template).
Figure 32 is an agarose gel electrophoresis photograph that shows the result of detecting *Vibrio parahaemolyticus* by using the 32^{nd} primer pair according to the present invention (M: DNA marker, *1: Staphylococcus aureus* (CCARM), 2: *Staphylococcus aureus* (NewMan), 3: *Staphylococcus aureus* (ATCC25923), 4: *Staphylococcus aureus* (ATCC23235), 5: *Yersinia enterocolitica, 6: Bacillus cereus, 7: Listeria monocytogenes, 8: Clostridium perfringens,* 9: EHEC, 10: EIEC, 11: *Salmonella typhimurium, 12: Vibrio vulnificus,* 13: *Vibrio cholerae,* 14: *Vibrio parahaemolyticus,* 15: *Campylobacter jejuni,* and 16: *Campylobacter coli*).

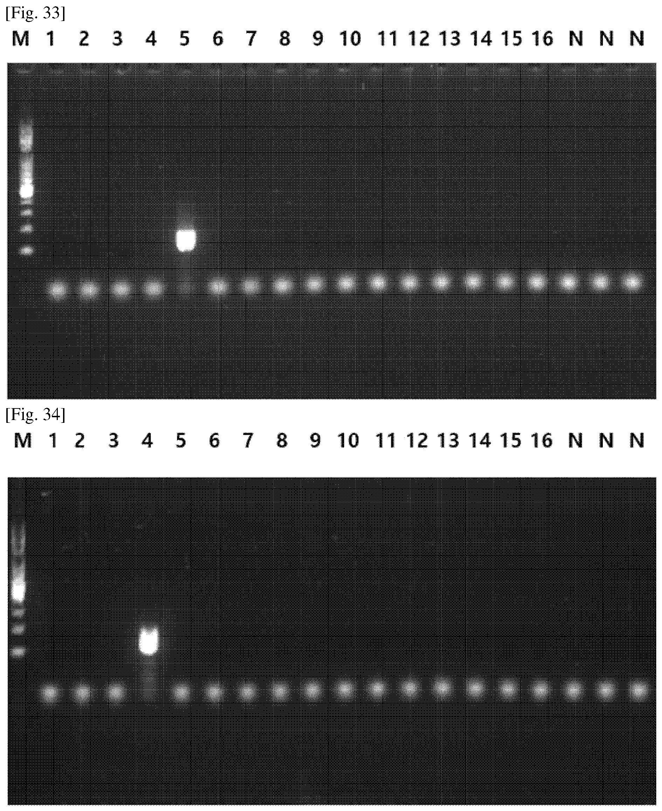

Figure 33 is an agarose gel electrophoresis photograph that shows the result of detecting *Vibrio parahaemolyticus* by using the 33^{rd} primer pair according to the present invention (M: DNA marker, *1: Campylobacter jejuni, 2: Campylobacter coli, 3: Clostridium perfringens, 4: Vibrio cholerae, 5: Vibrio parahaemolyticus, 6: Vibrio vulnificus, 7: Salmonella typhimurium, 8: Listeria monocytogenes, 9: Bacillus cereus, 10: Yersinia enterocolitica,* 11: *Staphylococcus aureus,* 12: EHEC, 13: ETEC, 14: EPEC, 15: EIEC, 16: EAEC, and N: no template).
Figure 34 is an agarose gel electrophoresis photograph that shows the result of detecting *Vibrio parahaemolyticus* by using the 34^{th} primer pair according to the present invention (M: DNA marker, *1: Campylobacter jejuni, 2: Campylobacter coli, 3: Clostridium perfringens, 4: Vibrio parahaemolyticus, 5: Vibrio vulnificus, 6: Vibrio cholerae, 7: Salmonella typhimurium, 8: Staphylococcus aureus, 9: Bacillus cereus, 10: Yersinia enterocolitica,* 11: *Listeria monocytogenes,* 12: EHEC, 13: ETEC, 14: EPEC, 15: EIEC, 16: EAEC, and N: no template).

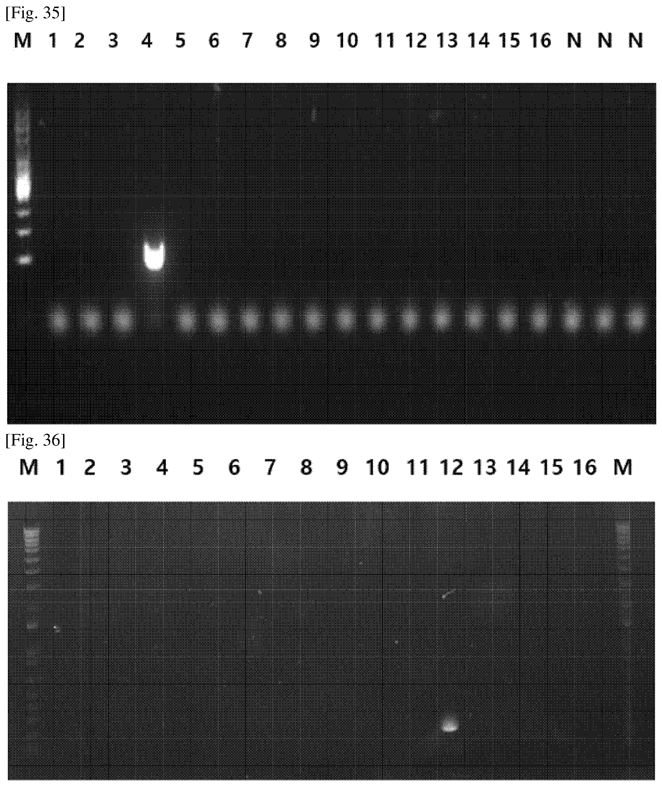

Figure 35 is an agarose gel electrophoresis photograph that shows the result of detecting *Vibrio parahaemolyticus* by using the 35^{th} primer pair according to the present invention (M: DNA marker, *1: Campylobacter jejuni, 2: Campylobacter coli, 3: Clostridium perfringens, 4: Vibrio parahaemolyticus, 5: Vibrio vulnificus, 6: Vibrio cholerae, 7: Salmonella typhimurium, 8: Staphylococcus aureus, 9: Bacillus cereus, 10: Yersinia enterocolitica,* 11: *Listeria monocytogenes,* 12: EHEC, 13: ETEC, 14: EPEC, 15: EIEC, 16: EAEC, and N: no template).
Figure 36 is an agarose gel electrophoresis photograph that shows the result of detecting *Vibrio vulnificus* by using the 36^{th} primer pair according to the present invention (M: DNA marker, *1: Staphylococcus aureus* (CCARM), 2: *Staphylococcus aureus* (NewMan), 3: *Staphylococcus aureus* (ATCC25923), 4: *Staphylococcus aureus* (ATCC23235), 5: *Yersinia enterocolitica, 6: Bacillus cereus, 7: Listeria monocytogenes, 8: Clostridium perfringens,* 9: EHEC, 10: EIEC, 11: *Salmonella typhimurium, 12: Vibrio vulnificus,* 13: *Vibrio cholerae,* 14: *Vibrio parahaemolyticus, 15: Campylobacter jejuni,* and 16: *Campylobacter coli*).

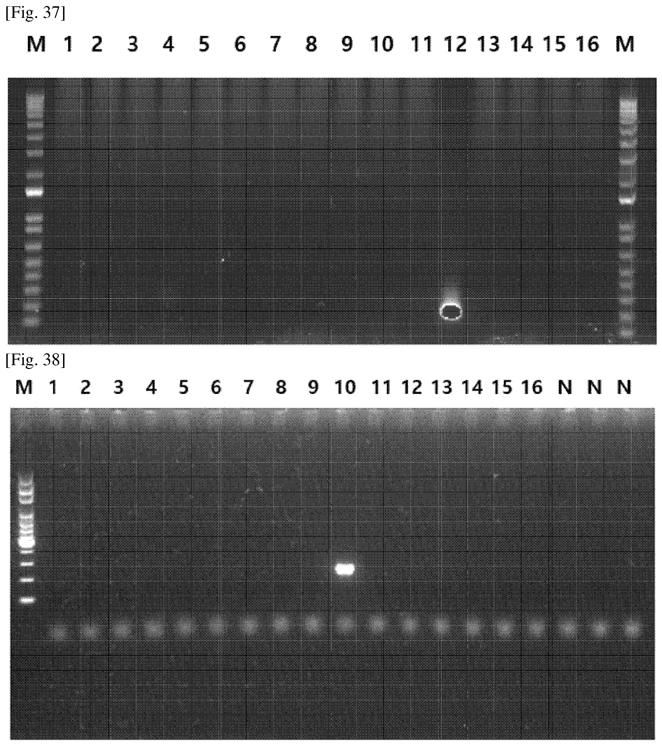

Figure 37 is an agarose gel electrophoresis photograph that shows the result of detecting *Vibrio vulnificus* by using the 37^{th} primer pair according to the present invention (M: DNA marker, *1: Staphylococcus aureus* (CCARM), 2: *Staphylococcus aureus* (NewMan), 3: *Staphylococcus aureus* (ATCC25923), 4: *Staphylococcus aureus* (ATCC23235), 5: *Yersinia enterocolitica, 6: Bacillus cereus, 7: Listeria monocytogenes, 8: Clostridium perfringens,* 9: EHEC, 10: EIEC, 11: *Salmonella typhimurium, 12: Vibrio vulnificus,* 13: *Vibrio cholerae,* 14: *Vibrio parahaemolyticus, 15: Campylobacter jejuni,* and 16: *Campylobacter coli).*
Figure 38 is an agarose gel electrophoresis analysis photograph that shows the result of detecting *Yersinia enterocolitica* by using the 38^{th} primer pair according to the present invention (M: DNA marker, *1: Campylobacter jejuni, 2: Campylobacter coli, 3: Clostridium perfringens, 4: Vibrio cholerae, 5: Vibrio parahaemolyticus, 6: Vibrio vulnificus, 7: Salmonella typhimurium, 8: Listeria monocytogenes, 9: Bacillus cereus, 10: Yersinia enterocolitica,* 11: *Staphylococcus aureus,* 12: EHEC, 13: EAEC, 14: EPEC, 15: EIEC, 16: ETEC, and N: no template).

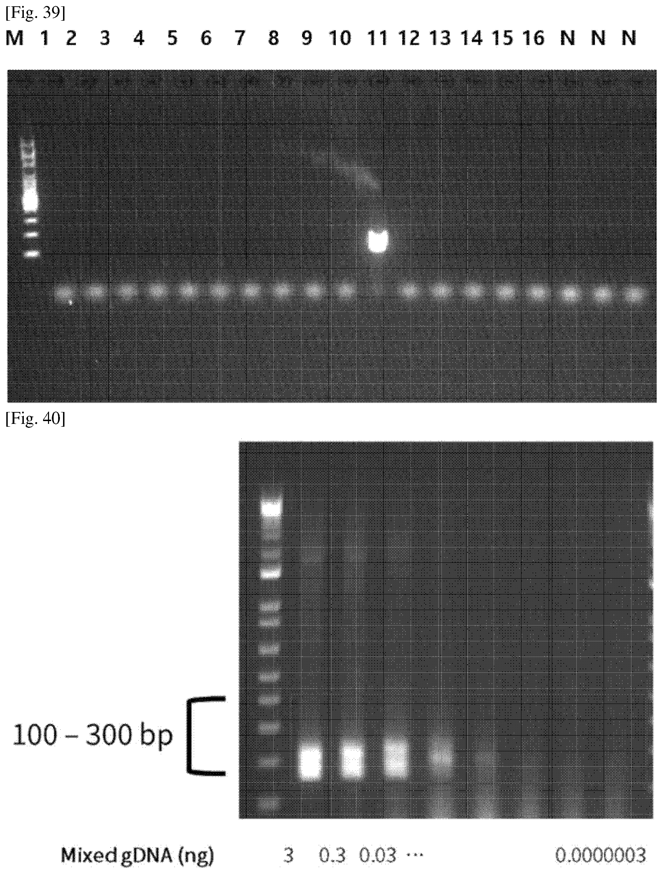

Figure 39 is an agarose gel electrophoresis analysis photograph that shows the result of detecting *Yersinia enterocolitica* by using the 39^{th} primer pair according to the present invention (M: DNA marker, *1: Campylobacter jejuni, 2: Campylobacter coli, 3: Clostridium perfringens, 4: Vibrio cholerae, 5: Vibrio parahaemolyticus, 6: Vibrio vulnificus, 7: Salmonella typhimurium, 8: Listeria monocytogenes, 9: Bacillus cereus, 10: Staphylococcus aureus,* 11: *Yersinia enterocolitica,* 12: EHEC, 13: ETEC, 14: EPEC, 15: EIEC, 16: EAEC, and N: no template).
Figure 40 is a result of performing a PCR, in accordance with an embodiment of the present invention, by mixing all primer pairs according to the present invention and food poisoning bacteria genes to perform NGS.

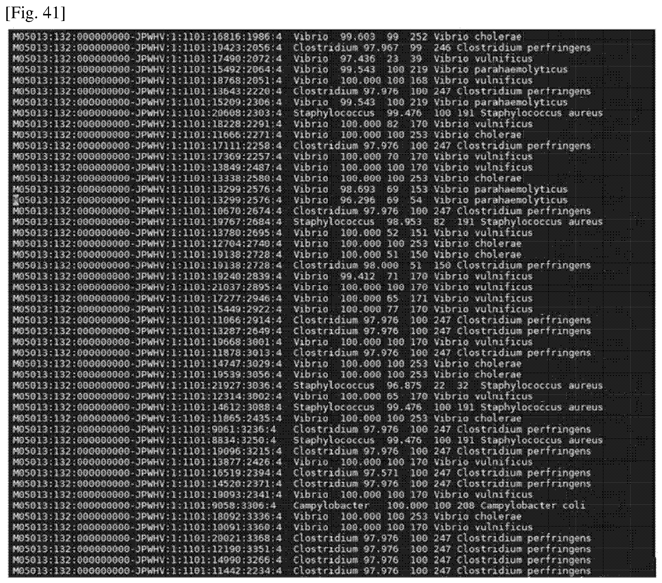

Figure 41 is a view showing a portion of the resulting data obtained by performing NGS according to an embodiment of the present invention.

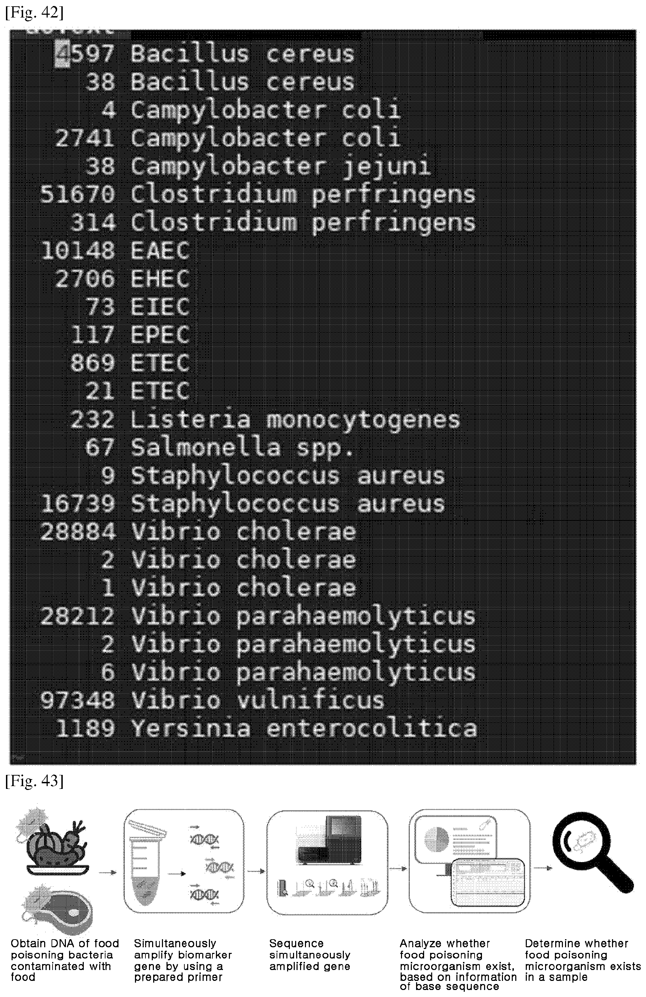

Figure 42 is a view showing the number of target sites detected by performing NGS according to an embodiment of the present invention.
Figure 43 is a schematic view showing a food poisoning bacteria detection method according to an embodiment of the present invention.

### Best Mode for Carrying out the Invention

Hereinafter, the present invention will be described in detail.

The present invention provides a primer set for detecting food poisoning bacteria as defined in the appendant claims.

The term 'food poisoning bacteria' used herein refers to bacteria that may cause, via food or water, intoxication symptoms such as acute gastroenteritis and neurological disorders. The food poisoning bacteria may include both bacteria (toxin-producing bacteria) that can grow in food and produce a toxin causing food poisoning and bacteria that can, when it is taken into a subject, cause food poisoning.

The food poisoning bacteria may include all kinds of food poisoning bacteria known in the ordinary art, and it may be, for example, at least one selected from the group consisting of *Bacillus, Campylobacter, Clostridium, Escherichia, Listeria, Salmonella, Staphylococcus, Vibrio,* and *Yersinia.* Specifically, the food poisoning bacteria may be at least one selected from the group consisting of *Bacillus cereus, Campylobacter coli, Campylobacter jejuni, Clostridium perfringens,* enteroaggregative *Escherichia coli* (EAEC), enterohemorrhagic *Escherichia coli* (EHEC), enteropathogenic *Escherichia coli* (EPEC), enteroinvasive *Escherichia coli* (EIEC), enterotoxigenic *Escherichia coli* (ETEC), *Listeria monocytogenes, Salmonella typhimurium, Staphylococcus aureus, Vibrio cholerae, Vibrio parahaemolyticus, Vibrio vulnificus* and *Yersinia enterocolitica.*

For example, the primer set as disclosed but not part of the invention may comprise:
i) at least one primer pair selected from the group consisting of the 1^{st} primer pair consisting of a base sequence represented by SEQ ID NO: 1 or 2 and the 2^{nd} primer pair consisting of a base sequence represented by SEQ ID NO: 3 or 4, the at least one primer pair being capable of detecting *Bacillus cereus;*
ii) the 3^{rd} primer pair consisting of a base sequence represented by SEQ ID NO: 5 or 6, the 3^{rd} primer pair being capable of detecting *Campylobacter;*
iii) at least one primer pair selected from the group consisting of the 4^{th} primer pair consisting of a base sequence represented by SEQ ID NO: 7 or 8 and the 5^{th} primer pair consisting of a base sequence represented by SEQ ID NO: 9 or 10, the at least one primer pair being capable of detecting *Campylobacter coli;*
iv) at least one primer pair selected from the group consisting of the 6^{th} primer pair consisting of a base sequence represented by SEQ ID NO: 11 or 12 and the 7^{th} primer pair consisting of a base sequence represented by SEQ ID NO: 13 or 14, the at least one primer pair being capable of detecting *Campylobacter jejuni;*
v) at least one primer pair selected from the group consisting of the 8^{th} primer pair consisting of a base sequence represented by SEQ ID NO: 15 or 16, the 9^{th} primer pair consisting of a base sequence represented by SEQ ID NO: 17 or 18, and the 10^{th} primer pair consisting of a base sequence represented by SEQ ID NO: 19 or 20, the at least one primer pair being capable of detecting *Clostridium perfringens;*
vi) the 11^{th} primer pair consisting of a base sequence represented by SEQ ID NO: 21 or 22, the 11^{th} primer pair being capable of detecting enteroaggregative *Escherichia coli* (EAEC);
vii) at least one primer pair selected from the group consisting of the 12^{th} primer pair consisting of a base sequence represented by SEQ ID NO: 23 or 24, the 13^{th} primer pair consisting of a base sequence represented by SEQ ID NO: 25 or 26, and the 14^{th} primer pair consisting of a base sequence represented by SEQ ID NO: 27 or 28, the at least one primer pair being capable of detecting enterohemorrhagic *Escherichia coli* (EHEC);
viii) the 15^{th} primer pair consisting of a base sequence represented by SEQ ID NO: 29 or 30, the 15^{th} primer pair being capable of detecting enteropathogenic *Escherichia coli* (EPEC);
ix) at least one primer pair selected from the group consisting of the 16^{th} primer pair consisting of a base sequence represented by SEQ ID NO: 31 or 32 and the 17^{th} primer pair consisting of a base sequence represented by SEQ ID NO: 33 or 34, the at least one primer pair being capable of detecting enteroinvasive *Escherichia coli* (EIEC);
x) the 18^{th} primer pair consisting of a base sequence represented by SEQ ID NO: 35 or 36, the 18^{th} primer pair being capable of detecting enterotoxigenic *Escherichia coli* (ETEC);
xi) at least one primer pair selected from the group consisting of the 19^{th} primer pair consisting of a base sequence represented by SEQ ID NO: 37 or 38, the 20^{th} primer pair consisting of a base sequence represented by SEQ ID NO: 39 or 40, and the 21^{st} primer pair consisting of a base sequence represented by SEQ ID NO: 41 or 42, the at least one primer pair being capable of detecting *Listeria monocytogenes;*
xii) at least one primer pair selected from the group consisting of the 22^{nd} primer pair consisting of a base sequence represented by SEQ ID NO: 43 or 44 and the 23^{rd} primer pair consisting of a base sequence represented by SEQ ID NO: 45 or 46, the at least one primer pair being capable of detecting *Salmonella typhimurium;*
xiii) at least one primer pair selected from the group consisting of the 24^{th} primer pair consisting of a base sequence represented by SEQ ID NO: 47 or 48, the 25^{th} primer pair consisting of a base sequence represented by SEQ ID NO: 49 or 50, the 26^{th} primer pair consisting of a base sequence represented by SEQ ID NO: 51 or 52, the 27 ^{th} primer pair consisting of a base sequence represented by SEQ ID NO: 53 or 54, and the 28^{th} primer pair consisting of a base sequence represented by SEQ ID NO: 55 or 56, the at least one primer pair being capable of detecting *Staphylococcus aureus;*
xiv) at least one primer pair selected from the group consisting of the 29^{th} primer pair consisting of a base sequence represented by SEQ ID NO: 57 or 58, the 30^{th} primer pair consisting of a base sequence represented by SEQ ID NO: 59 or 60, and the 31^{st} primer pair consisting of a base sequence represented by SEQ ID NO: 61 or 62, the at least one primer pair being capable of detecting *Vibrio cholerae;*
xv) at least one primer pair selected from the group consisting of the 32^{nd} primer pair consisting of a base sequence represented by SEQ ID NO: 63 or 64, the 33^{rd} primer pair consisting of a base sequence represented by SEQ ID NO: 65 or 66, the 34^{th} primer pair consisting of a base sequence represented by SEQ ID NO: 67 or 68, and the 35^{th} primer pair consisting of a base sequence represented by SEQ ID NO: 69 or 70, the at least one primer pair being capable of detecting *Vibrio parahaemolyticus;*
xvi) at least one primer pair selected from the group consisting of the 36^{th} primer pair consisting of a base sequence represented by SEQ ID NO: 71 or 72 and the 37^{th} primer pair consisting of a base sequence represented by SEQ ID NO: 73 or 74, the at least one primer pair being capable of detecting *Vibrio vulnificus;* and
xvii) at least one primer pair selected from the group consisting of the 38^{th} primer pair consisting of a base sequence represented by SEQ ID NO: 75 or 76 and the 39^{th} primer pair consisting of a base sequence represented by SEQ ID NO: 77 or 78, the at least one primer pair being capable of detecting *Yersinia enterocolitica.*

Detection of food poisoning bacteria by using the primer set can be carried out by using a molecular detection method. The term 'molecular detection' refers to a method of detecting, by numerical values or images, changes occurring in cells at various molecular levels. The molecular detection generally refers to analysis of nucleic acids such as DNA or RNA, but it may include analysis of proteins and intracellular metabolom. Specifically, for example, the primer set according to the present invention can be used to detect food poisoning bacteria through DNA analysis.

The detection can be performed by amplifying a target gene present in a food poisoning bacteria genome. At this time, the amplification of the gene can be carried out in any way known in the art. For example, the gene amplification may be carried out by a polymerase chain reaction, a real-time polymerase chain reaction (real time PCR), or multiple polymerase chain reactions (multiplex PCR), and the like. In another aspect of the present invention, the detection can be carried out by a next-generation sequencing method.

The primer constituting the primer set may be a conjugate labeled with a detector such as a coloring enzyme, a fluorescent agent, a radioisotope, or a colloid. Examples of the coloring enzyme may include peroxidase, alkaline phosphatase, or acidic phosphatase. Examples of the fluorescent agent may include fluorescein thiourea (FTH), 7-acetoxycoumarin-3-yl, fluorescein-5-yl, fluorescein-6-yl, 2',7'-dichlorofluorescein-5-yl, 2',7'-dichlorofluorescein-6-yl, dihydrotetramethyl-losamine-4-yl, tetramethyllodamine-5-yl, tetramethyllodamine-6-yl, 4,4-difluoro-5,7-dimethyl-4-bora-3a, 4a-diaza-s-indacene-3- ethyl or 4,4-difluoro-5,7-diphenyl-4-bora-3a, 4a-diaza-s-indacene-3-ethyl, Cy3, Cy5, poly L-lysine-fluorescein iso thiocyanate (FITC), rhodamine-B-isothiocyanate (RITC), Phycoerythrin (PE) or rhodamine.

In addition, the present invention provides a composition for detecting food poisoning bacteria, the composition comprising the primer set.

The primers that may be included in the composition according to the present invention and the food poisoning bacteria that can be detected by using the primers may have the characteristics described above.

In addition, the composition may further comprise a ligand that can be specifically bound to the primer included in the primer set according to the present invention. The ligand may be a conjugate in which detectors such as coloring enzymes, fluorescent agents, radioisotopes, or colloids are labeled, and the ligand may be treated with streptavidin or avidin. The composition according to the present invention may further comprise distilled water or a buffer to ensure structural stability.

In addition, the present invention provides a next-generation sequencing panel for detecting food poisoning bacteria, the panel comprising the primer set.

The primers that may be included in the panel according to the present invention and the food poisoning bacteria that can be detected by using the primers may have the characteristics described above.

The term 'next-generation sequencing' or 'NGS' used herein refers to a method that can sequence bases of a gene at a high speed, in which a gene can be put into many pieces, the pieces can be read at the same time, and the thus-obtained data can be combined by using bioinformatic techniques, thereby making it possible to quickly decipher the genetic information. Examples of the next-generation sequencing may include single molecule real-time sequencing, ion semiconductor / ion torrent sequencing, pyrosequencing, sequencing by synthesis, sequencing by ligation, nanopore sequencing, and the like.

In addition, the present invention provides a kit for detecting food poisoning bacteria, the kit comprising the primer set.

The primers that may be included in the kit according to the present invention and the food poisoning bacteria that can be detected by using the primers may have the characteristics described above.

The kit may be coupled to a solid substrate to facilitate subsequent steps, such as washing the primer or dissembling the kit. Examples of the solid substrate may include synthetic resins, nitrocellulose, glass substrates, metal substrates, glass fibers, microspheres, and microbeads. In addition, examples of the synthetic resins may include polyesters, polyvinyl chlorides, polystyrenes, polypropylenes, PVDFs, and nylons.

In addition, the kit may be prepared by a conventional manufacturing method known in the art, and it may further comprise buffers, stabilizers, inert proteins, and the like. A high throughput screening (high throughput screening; HTS) system in which a fluorescence level of a fluorescent agent used as a detector is measured or the radiation level of a radiation isotope used as a detector is measured, a surface plasmon resonance (SPR) method in which a surface plasmon resonance change is measured in real time without using a detector, or a surface plasmon resonance imaging (SPRI) method in which data from the SPR system is converted to image data can be used.

The type of food poisoning bacteria that can be detected by using the kit is as described above. On the other hand, in another aspect of the present invention, a next-generation sequencing method may be used to detect food poisoning bacteria by using the kit. In order to detect food poisoning bacteria by the next-generation sequencing method, additional reagents commonly used in the next-generation sequencing may be further comprised the kit. For example, the kit may further comprise a reagent, such as a polymerase, dNTP, and the like, for amplifying a target site. In addition, the kit may further comprise an A-tail, an adapter, a bead, a buffer, and so on for purifying an amplified product and using the amplified product to prepare a library for us in next-generation sequencing. The additional reagents may be used appropriate quantities by an ordinary skilled person.

In addition, the present invention provides a next-generation sequencing method comprising the steps of amplifying a target sequence by using a sample and the primer set; preparing a library by using the amplified target sequence; and performing a next-generation sequencing analysis by using the prepared library.

The primers that may be used in the next-generation sequencing method according to the present invention and the food poisoning bacteria that can be detected by using the primers may have the characteristics described above.

On the other hand, the next-generation sequencing analysis may also have the characteristics as described above, which can be performed by any method known in the art. The method may be suitably modified by an ordinary skilled person in the art.

Any sample can be used for the purpose of detecting food poisoning bacteria. For example, the sample may be any food material such as vegetables, meat, fish, seafood, etc. or any food prepared by using the food materials. On the other hand, the sample may be used to diagnose a subject with food poisoning. The subject may be, for example, mammals, including humans.

In addition, before being applied to the detection method according to the present invention, the sample can be suitably pretreated by using various methods such as anion exchange chromatography, affinity chromatography, size exclusion chromatography, liquid chromatography, continuous extraction, centrifugation, gel electrophoresis, and so on.

Furthermore, the present invention provides a method for detecting food poisoning bacteria, the method comprising the step of amplifying by using the primer set and the sample.

The primers that may be used in the methods according to the present invention and the food poisoning bacteria that can be detected by using the primers may have the characteristics described above. The amplification may be performed in the same manner as described above. The amplification may be performed by using a reaction product prepared in a conventional manner. In one aspect of the present invention, the next-generation sequencing method can be used, which sequencing method can be carried out in a conventional manner. In addition, this method can be appropriately modified by an ordinary skilled person in the art.

The sample used in the detection methods according to the present invention may have the characteristics as described above, and the sample may be pretreated as described above.

### Mode for the Invention

Hereinafter, the present invention will be described in detail by the following embodiments, but, the present invention is not limited to the embodiments as the embodiments are only intended to illustrate the present invention. Any subject matter that has the same structure as the subject matter claimed in the following claim section and have the same effect as the effect resulting from the subject matter claimed in the following claim section should be included in the technical scope of the present invention.

### Example 1. Preparation of Primer Set

A primer set for simultaneously detecting 16 types of food poisoning bacteria by using next-generation sequencing (NGS) was prepared.

First, from the gene sequences of 16 types of food poisoning bacteria, a region that has the highest homology within the same species of food poisoning bacteria and does not have homology between different species of food poisoning bacteria was selected, and a primer corresponding to the selected region was prepared. The primer comprised 20 to 23 bases, and the primer was prepared such that the PCR product obtained through PCR amplification was 150 to 250 bp long, had GC content of 40 to 60%, and had a Tm value of 53 to 60°C. In addition, the self-compatibility was at least 4. The prepared primer set is shown in Table 1 below.

**[Table 1]**

| strains | primer | sequence | SEQ ID NO. |
|---|---|---|---|
| *Bacillus cereus* | 1-F | GCG CTC TTC TAA AGT CTC AC | SEQ ID NO: 1 |
| | 1-R | CGA AAT TAG CCC AGT AGC AC | SEQ ID NO: 2 |
| | 2-F | GAA CTG CTG GTA CAA CAC CTG | SEQ ID NO: 3 |
| | 2-R | TCT GCA CTA ATG AAC TGA CCG | SEQ ID NO: 4 |
| *Campylobacter* spp. | 3-F | CCG CTT TTG GAC CTC AAT CTC GC | SEQ ID NO: 5 |
| | 3-R | GCC TTG TGC GCG TTC TTT ATT GCC | SEQ ID NO: 6 |
| *Campylobacter coli* | 4-F | GCA AGA ATT ACC TTT GGT TGA | SEQ ID NO: 7 |
| | 4-R | CCT CCA TTT GCT TGT ATT CAT | SEQ ID NO: 8 |
| | 5-F | CCT ACG ACT TGT TTG ATT TGG | SEQ ID NO: 9 |
| | 5-R | CCA CAG AAT GAC CTC CAT TAG | SEQ ID NO: 10 |
| *Campylobacter jejuni* | 6-F | TGG AGT TGT TGT GGG GCT TC | SEQ ID NO: 11 |
| | 6-R | TGA TGC GTT CTT TTG CAC CC | SEQ ID NO: 12 |
| | 7-F | TTA TGG CGA GGG ACT TGG AGC | SEQ ID NO: 13 |
| | 7-R | AGA ATA ATG CGC CGT TGG ACG | SEQ ID NO: 14 |
| *Clostridium perfringens* | 8-F | ATG TTA CTG CCG TTG ATA GCG | SEQ ID NO: 15 |
| | 8-R | GCT GCA TAA TCC CAA TCA TCC C | SEQ ID NO: 16 |
| | 9-F | GGC GTT CTT CTA ACT CAT ACC C | SEQ ID NO: 17 |
| | 9-R | CTC CAT CAC CTA AGG ACT GTT C | SEQ ID NO: 18 |
| | 10-F | GCC TTA TTG TCT TAT TGG TCT | SEQ ID NO: 19 |
| | 10-R | GAA AAA TGG AAT AGC ACC TAA | SEQ ID NO: 20 |
| EAEC | 11-F | GAT GCT GAC GAT TCT GTA TTA | SEQ ID NO: 21 |
| | 11-R | ATA AGT CCT TCT CGA TTG TGT | SEQ ID NO: 22 |
| EHEC | 12-F | GAT AGA TCC AGA GGA AGG GCG | SEQ ID NO: 23 |
| | 12-R | TAC GAC TGA TCC CTG CAA CAC | SEQ ID NO: 24 |
| | 13-F | ACT GTC TGA AAC TGC TCC TGT | SEQ ID NO: 25 |
| | 13-R | GGT TGA CTC TCT TCA TTC ACG | SEQ ID NO: 26 |
| | 14-F | CTC AGG ATG CTA AAC CAG TAG AG | SEQ ID NO: 27 |
| | 14-R | CCG GTA CAA GCA GGA TTA CAA C | SEQ ID NO: 28 |
| EPEC | 15-F | TAG TGG ATT GGA CTC AAC GAT | SEQ ID NO: 29 |
| | 15-R | TAT TAA CAC CGT AGC CTT TCG | SEQ ID NO: 30 |
| EIEC | 16-F | ACG AGT CAA CTT TTA GCG AAG GG | SEQ ID NO: 31 |
| | 16-R | CTC TAT TTC CAA TCG CGT CAG AAC | SEQ ID NO: 32 |
| | 17-F | TCC TGC TTA GAT GAT GGA GG | SEQ ID NO: 33 |
| | 17-R | CCA AAA GGA AGT GTC TGC TC | SEQ ID NO: 34 |
| ETEC | 18-F | TGA CGG ATA TGT TTC CAC TTC | SEQ ID NO: 35 |
| | 18-R | GTA TTC CAC CTA ACG CAG AAA | SEQ ID NO: 36 |
| *Listeria monocytogenes* | 19-F | CTG GTG ATA CTC TTT GGG GTA | SEQ ID NO: 37 |
| | 19-R | AGC CGT TAG ATT CGG TTG TTT C | SEQ ID NO: 38 |
| | 20-F | GTG ACG AAG TAG AAG TTA TCG | SEQ ID NO: 39 |
| | 20-R | AGT TAG TGT GTG GAG TAA TCG | SEQ ID NO: 40 |
| | 21-F | TTG ATG GTG CTG TTG CGG TTC | SEQ ID NO: 41 |
| | 21-R | TGG GAG TTG GAT TGG GTG C | SEQ ID NO: 42 |
| *Salmonella typhimurium* | 22-F | CGC ACT GAA TAT CGT ACT GGA | SEQ ID NO: 43 |
| | 22-R | CGA TAA TTT CAC CGG CAT CG | SEQ ID NO: 44 |
| | 23-F | GCT GAG TAA CCA ACA AGA TAA | SEQ ID NO: 45 |
| | 23-R | AGT AAA CGC TGT TCA TAG GTC | SEQ ID NO: 46 |
| *Staphylococcus aureus* | 24-F | GCA GCT TGC TTA CTT ACT GCT | SEQ ID NO: 47 |
| | 24-R | TAC CTG TAA TCT CGC CAT CAT | SEQ ID NO: 48 |
| | 25-F | ATT CAT TGC CCT AAC GTG GAC | SEQ ID NO: 49 |
| | 25-R | GCT GTA AAA ATT GAT CGT GAC TCT C | SEQ ID NO: 50 |
| | 26-F | GTA TGG TGG TGT AAC TGA GC | SEQ ID NO: 51 |
| | 26-R | CCG TTT CAT AAG GCG AGT TG | SEQ ID NO: 52 |
| | 27-F | CTG CTA TTT TTC ATC CAA AGA | SEQ ID NO: 53 |
| | 27-R | TTC TTA TCA GTT TGC ACT TCA | SEQ ID NO: 54 |
| | 28-F | TGT CAC TCC ACA CGA AGG TA | SEQ ID NO: 55 |
| | 28-R | TGC AAA TAG CGC CTT GCT TG | SEQ ID NO: 56 |
| *Vibrio cholerae* | 29-F | GCC AAG AGG ACA GAG TGA GTA | SEQ ID NO: 57 |
| | 29-R | ATG AGG ACT GTA TGC CCC TA | SEQ ID NO: 58 |
| | 30-F | TAA GAC CAA CAG CAA CCG CCC | SEQ ID NO: 59 |
| | 30-R | ACT CGA CTG GCG TAA CCA AAA GG | SEQ ID NO: 60 |
| | 31-F | GTT TGT ATG TGC GAG CGG GTG | SEQ ID NO: 61 |
| | 31-R | GTG AAT GTC AGC GCC ACC AAC | SEQ ID NO: 62 |
| *Vibrio parahaemolyticus* | 32-F | GCA CTG TCT CAT TTC CCA GAG | SEQ ID NO: 63 |
| | 32-R | CGC TTC TTG GTC AGA AAC CAG | SEQ ID NO: 64 |
| | 33-F | ACC TGT GGC TTC TGC TGT G | SEQ ID NO: 65 |
| | 33-R | CCA GTT GTT GAT TTG CGG GTG | SEQ ID NO: 66 |
| | 34-F | TCC ATC TGT CCC TTT TCC TGC C | SEQ ID NO: 67 |
| | 34-R | CAG CCA TTT AGT ACC TGA CGT TGT G | SEQ ID NO: 68 |
| | 35-F | GCG AGC GAT CCT TGT TTG GAC | SEQ ID NO: 69 |
| | 35-R | GCG GTG AGT TGC TGT TGT TGG | SEQ ID NO: 70 |
| *Vibrio vulnificus* | 36-F | CTC TGC CTA GAT GTT TAT GG | SEQ ID NO: 71 |
| | 36-R | CAA TAC CAT TTC TGT GCT AAG | SEQ ID NO: 72 |
| | 37-F | AGC ACA TCT CTA TTC CTT CTC | SEQ ID NO: 73 |
| | 37-R | TAG CGT TGC TTC TTC AGT AA | SEQ ID NO: 74 |
| *Yersinia enterocolitica* | 38-F | GG GGC CAT CTT TCC GCA TTA | SEQ ID NO: 75 |
| | 38-R | TAC CCT GCA CCA AGC ATC CAA | SEQ ID NO: 76 |
| | 39-F | AAC GGG GCA TCT GGT TCT CTC | SEQ ID NO: 77 |
| | 39-R | TGG TGG TGT CAG GAA AGG GAC | SEQ ID NO: 78 |

### Example 2. Identification of Detectability of Primer

PCR was performed to identify whether the prepared primers specifically detect respective target bacteria.

Specifically, using each gene of the 16 types of food poisoning bacteria as a template, whether each of the 1^{st} to 39^{th} primer sets specifically detects certain target bacteria was identified. The strains used were *Bacillus cereus* (Sanigen, Korea), *Campylobacter coli* (Sanigen, Korea), *Campylobacter jejuni* (Sanigen, Korea), *Clostridium perfringens* (FORC_25), enteroaggregative *Escherichia coli* (EAEC)(NCCP14039), enterohaemorrhagic *Escherichia coli* (EHEC) (Sanigen, Korea), enteropathogenic *Escherichia coli* (EPEC) (Sanigen, Korea), enteroinvasive *Escherichia coli* (EIEC) (Sanigen, Korea), enterotoxigenic *Escherichia coli* (ETEC) (Sanigen, Korea), *Listeria monocytogenes* (Sanigen, Korea), *Salmonella typhimurium* (Sanigen, Korea), *Staphylococcus aureus* (ATCC25929, ATCC23235, CCARM3089, Newman), *Vibrio cholerae* (Sanigen, Korea), *Vibrio parahaemolyticus* (Sanigen, Korea), *Vibrio vulnificus* (Sanigen, Korea), and *Yersinia enterocolitica* (Sanigen, Korea). The gene templates were prepared by using Nucleospin^{®} Microbial DNA kit (Macherey-Nagel) in accordance with the manufacturer's protocol. 1 *µℓ* of template DNA, 0.1 *µℓ* of a forward primer, 0.1 *µℓ* of a reverse primer, 10 *µℓ* of master mix (Biofact), and 8.8 *µℓ* of distilled water were mixed to obtain a PCR reactant. With the PCR reactant, PCR was performed under the conditions described in Table 2 below, and the obtained PCR product was confirmed by using an agarose gel. The results of the agarose gel electrophoresis are shown in Figures 1 to 39.

**[Table 2]**

| step | temperature | time | cycle |
|---|---|---|---|
| pre-denaturation | 95°C | 4 minutes | 1 time |
| denaturation | 95°C | 30 seconds | 35 times |
| annealing | 60°C | 30 seconds | |
| extension | 72°C | 30 seconds | |
| post-extension | 72°C | 5 minutes | 1 time |

As shown in Figures 1 to 39, the primer sets of 1^{st} to 39^{th} specifically detected respective target food poisoning bacteria.

### Example 3. Detection of Food Poisoning Bacteria by using NGS

### 3-1. Amplification of Target Sequence

Both the primers described in Table 1 and the genes of the 16 types of food poisoning bacteria were mixed, and PCR was performed to amplify the target sequences. First, a template DNA mixture was prepared by mixing the DNAs of the 16 strains to be 3 ng, and a forward or reverse primer was prepared by mixing the primers listed in Table 1 to be 10 pmol. Thereafter, 2.5 *µℓ* of template DNA mixture, 5 *µ*ℓ of a forward primer mixture, 5 *µ*ℓ of a reverse primer mixture, and 12.5 *µℓ* of HIFI KAPA hyperflus master mix (Roche) were mixed to obtain a PCR reactant. With the PCR reactant, PCR was performed under the conditions described in Table 2 below, and the obtained PCR product was confirmed by using an agarose gel. The results of the agarose gel electrophoresis are shown in Figure 40.

As shown in Figure 40, PCR products having a size in the range of 150 to 250 bp were prepared as a result of the amplification by the 39 primer sets described in Table 1.

### 3-2. Preparation of Library

Using the thus-obtained PCR products, a library was prepared as follows.

First, 50 *µ*ℓ of a sample purification bead (SPB, Illumina) and 25 *µℓ* of the PCR products were mixed well on the tube and incubated for 5 minutes at room temperature. After the incubation, the tube was transferred to the magnetic stand and incubated further for 3 minutes until the liquid was clear, and the supernatant was removed. The remained bead was washed by adding 200 *µℓ* of 80% ethanol. The mixture was incubated on the magnetic stand for 30 seconds. After then, the supernatant of ethanol was removed. This process was repeated twice to wash the beads and completely remove the ethanol. 62.5 *µℓ* of a resuspension buffer (Illumina) and the washed bead were mixed, and the mixture was incubated for 2 minutes. The mixture was transferred to the magnetic stand and incubated for a further 3 minutes, and then 60 *µ*ℓ of the supernatant was taken and transferred to a new tube. 40 *µℓ* of an end repair mix2 (Illumina) was added to the new tube, and the mixture was reacted for 30 minutes at 30°C to repair incomplete ends. After the reaction was finished, the reactant and 160 *µℓ* of SPB were mixed well, and the mixture was incubated for 5 minutes at room temperature. The mixture was transferred to the magnetic stand and incubated for a further 5 minutes, and the supernatant was removed. Thereafter, the bead was washed by ethanol, a resuspension buffer was added to the washed bead, and 17.5 *µℓ* of the supernatant was taken. The 17.5 *µℓ* of the supernatant and 12.5 *µℓ* of A-tailing mix (Illumina) was mixed and reacted for 30 minutes at 97°C and for 5 minutes at 72°C. 30 *µℓ* of the resulting reactant, 2.5 *µ*ℓ of a ligation mix (Illumina), TruSeq DNA CD indexes (Illumina) and 2.5 *µℓ* of a resuspension buffer were mixed. The mixture was reacted at 30°C for 10 minutes, and 5 *µℓ* of a stop ligation buffer (Illumina) was added to terminate the reaction. 42.5 *µℓ* of the reaction product, 46 *µℓ* of sample purification bead, and 46 *µℓ* of deionized water were mixed well, and the mixture was incubated for 5 minutes at room temperature. Only the supernatant was taken and placed in a new tube. 105 *µℓ* of sample purification bead was added, and the mixture was incubated at room temperature for 5 minutes. Thereafter, the bead was washed with ethanol as described above, and the process of adding a resuspension buffer to the washed bead was repeated twice to obtain 25 *µℓ* of a library.

### 3-3. Library Amplification

The thus-prepared library was amplified as follows.

Specifically, 25 *µℓ* of the library, 20 *µℓ* of enhanced PCR mix (Illumina), 5 *µℓ* of the primer mixture prepared in Example 3-1 were mixed to obtain a PCR reactant. With the PCR reactant, PCR was performed under the conditions as described in Table 3 below.

**[Table 3]**

| step | temperature | time | cycle |
|---|---|---|---|
| pre-denaturation | 98°C | 3 minutes | 1 time |
| denaturation | 98°C | 20 seconds | 12 times |
| annealing | 60°C | 15 seconds | |
| extension | 72°C | 30 seconds | |
| post-extension | 72°C | 5 minutes | 1 time |

The thus-obtained PCR product and 50 *µℓ* of sample purification bead were mixed, the mixture was incubated and washed as described above, and a resuspension buffer was added to obtain 30 *µℓ* of a PCR product.

### 3-4. Measurement of Concentration of Final Product and Performance of NGS

The concentration of the PCR product obtained above was measured by using a bio-analyzer, and the concentration was adjusted to be 4 nM. 4nM of the PCR product was diluted to 7 pM, and the diluted PCR product and a phiX solution (Illumina) were mixed with a volume ratio of 7:3. With the mixture, NGS was performed by Miseq NGS equipment according to the manufacturer's protocol. Data obtained through NGS was analyzed by using Trimmomatic, Pandaseq, and BLAST. Specifically, the read values obtained by the Miseq NGS equipment was filtered based on the phred score of 15, and the values were summed by read unit. On the other hand, by a BLAST analysis of the base sequence of the target region amplified by the primers was filtered based on 95% homology. In this process, the final result was achieved by counting the number of matching read values. A portion of the analysis data is shown in Figure 41, the result of combining the number of target sites detected is shown in Figure 42, and analysis data with respect to the strains is shown in Table 4.

**[Table 4]**

| strain | target location | amplicon size (bp) | 3.E+00 | 3.E-01 | 3.E-02 | 3.E-03 |
|---|---|---|---|---|---|---|
| *Bacillus cereus* | 1 | 175 | 857 | 361 | 490 | 296 |
| | 2 | 229 | 6426 | 3881 | 2373 | 2177 |
| *Campylobacter* spp. | 3 | 191 | 608 | 194 | 290 | 173 |
| *Campylobacter coli* | 4 | 203 | 6179 | 6389 | 11298 | 13502 |
| | 5 | 208 | 8514 | 7689 | 7722 | 9281 |
| *Campylobacter jejuni* | 6 | 184 | 2390 | 265 | 129 | 38 |
| | 7 | 163 | 8682 | 7046 | 11693 | 8354 |
| *Clostridium perfringens* | 8 | 247 | 10990 | 9645 | 8051 | 14150 |
| | 9 | 168 | 3820 | 2427 | 11369 | 9258 |
| | 10 | 178 | 2890 | 3322 | 9173 | 13574 |
| EAEC | 11 | 187 | 21964 | 21054 | 24069 | 38173 |
| EHEC | 12 | 209 | 26 | 17 | 7 | 2 |
| | 13 | 255 | 10858 | 7945 | 10576 | 8052 |
| | 14 | 154 | 1967 | 832 | 673 | 413 |
| EPEC | 15 | 233 | 12399 | 6306 | 5858 | 3006 |
| EIEC | 16 | 234 | 8703 | 4226 | 1938 | 3196 |
| | 17 | 173 | 635 | 201 | 84 | 36 |
| ETEC | 18 | 191 | 2640 | 3691 | 8497 | 8303 |
| *Listeria monocytogenes* | 19 | 264 | 5269 | 1296 | 1055 | 743 |
| | 20 | 198 | 2488 | 683 | 105 | 50 |
| | 21 | 200 | 26 | 1 | 0 | 1 |
| *Salmonellatyphimuri um* | 22 | 176 | 1620 | 1092 | 2272 | 714 |
| | 23 | 186 | 4893 | 2172 | 549 | 336 |
| *Staphylococcus ureus* | 24 | 214 | 914 | 2172 | 3478 | 518 |
| | 25 | 191 | 14317 | 12622 | 22756 | 54297 |
| | 26 | 212 | 1480 | 1539 | 838 | 818 |
| | 27 | 180 | 1203 | 2015 | 831 | 893 |
| | 28 | 162 | 2054 | 768 | 10 | 278 |
| *Vibrio cholerae* | 29 | 253 | 5067 | 3656 | 5803 | 5795 |
| | 30 | 209 | 2955 | 987 | 325 | 223 |
| | 31 | 175 | 319 | 90 | 21 | 3 |
| *Vibrio parahaemolyticus* | 32 | 219 | 3832 | 2287 | 9705 | 7423 |
| | 33 | 178 | 2814 | 837 | 312 | 79 |
| | 34 | 190 | 1499 | 126 | 4 | 13 |
| | 35 | 144 | 65 | 18 | 23 | 31 |
| *Vibrio vulnificus* | 36 | 218 | 4557 | 851 | 52 | 103 |
| | 37 | 170 | 11946 | 13190 | 31663 | 42789 |
| *Yersinia enterocolitica* | 38 | 202 | 20 | 8 | 1 | 2 |
| | 39 | 190 | 4348 | 3257 | 3187 | 1204 |

As shown in Table 4, 16 types of food poisoning bacteria were detected at once by the NGS method by using the primers according to the present invention.

## Claims

1. A primer set for detecting food poisoning bacteria, the primer set comprising:
a) at least one primer pair selected from the group consisting of the 1st primer pair consisting of a base sequence represented by SEQ ID NO: 1 and 2, respectively and the 2nd primer pair consisting of a base sequence represented by SEQ ID NO: 3 and 4, respectively;
b) the 3rd primer pair consisting of a base sequence represented by SEQ ID NO: 5 and 6, respectively;
c) at least one primer pair selected from the group consisting of the 4th primer pair consisting of a base sequence represented by SEQ ID NO: 7 and 8, respectively and the 5th primer pair consisting of a base sequence represented by SEQ ID NO: 9 and 10, respectively;
d) at least one primer pair selected from the group consisting of the 6th primer pair consisting of a base sequence represented by SEQ ID NO: 11 and 12, respectively and the 7th primer pair consisting of a base sequence represented by SEQ ID NO: 13 and 14, respectively;
e) at least one primer pair selected from the group consisting of the 8th primer pair consisting of a base sequence represented by SEQ ID NO: 15 and 16, respectively, the 9th primer pair consisting of a base sequence represented by SEQ ID NO: 17 and 18, respectively, and the 10th primer pair consisting of a base sequence represented by SEQ ID NO: 19 and 20, respectively;
f) the 11^{th} primer pair consisting of a base sequence represented by SEQ ID NO: 21 and 22, respectively;
g) at least one primer pair selected from the group consisting of the 12th primer pair consisting of a base sequence represented by SEQ ID NO: 23 and 24, respectively, the 13th primer pair consisting of a base sequence represented by SEQ ID NO: 25 and 26, respectively, and the 14th primer pair consisting of a base sequence represented by SEQ ID NO: 27 and 28, respectively;
h) the 15th primer pair consisting of a base sequence represented by SEQ ID NO: 29 and 30, respectively;
i) at least one primer pair selected from the group consisting of the 16th primer pair consisting of a base sequence represented by SEQ ID NO: 31 and 32, respectively and the 17th primer pair consisting of a base sequence represented by SEQ ID NO: 33 and 34, respectively;
j) the 18th primer pair consisting of a base sequence represented by SEQ ID NO: 35 and 36, respectively;
k) at least one primer pair selected from the group consisting of the 19th primer pair consisting of a base sequence represented by SEQ ID NO: 37 and 38, respectively, the 20th primer pair consisting of a base sequence represented by SEQ ID NO: 39 and 40, respectively, and the 21 st primer pair consisting of a base sequence represented by SEQ ID NO: 41 and 42, respectively;
l) at least one primer pair selected from the group consisting of the 22nd primer pair consisting of a base sequence represented by SEQ ID NO: 43 and 44, respectively and the 23rd primer pair consisting of a base sequence represented by SEQ ID NO: 45 and 46, respectively;
m) at least one primer pair selected from the group consisting of the 24th primer pair consisting of a base sequence represented by SEQ ID NO: 47 and 48, respectively, the 25th primer pair consisting of a base sequence represented by SEQ ID NO: 49 and 50, respectively, the 26th primer pair consisting of a base sequence represented by SEQ ID NO: 51 and 52, respectively, the 27th primer pair consisting of a base sequence represented by SEQ ID NO: 53 and 54, respectively, and the 28th primer pair consisting of a base sequence represented by SEQ ID NO: 55 and 56, respectively;
n) at least one primer pair selected from the group consisting of the 29th primer pair consisting of a base sequence represented by SEQ ID NO: 57 and 58, respectively, the 30th primer pair consisting of a base sequence represented by SEQ ID NO: 59 and 60, respectively, and the 31st primer pair consisting of a base sequence represented by SEQ ID NO: 61 and 62, respectively;
o) at least one primer pair selected from the group consisting of the 32nd primer pair consisting of a base sequence represented by SEQ ID NO: 63 and 64, respectively, the 33rd primer pair consisting of a base sequence represented by SEQ ID NO: 65 and 66, respectively, the 34th primer pair consisting of a base sequence represented by SEQ ID NO: 67 and 68, respectively, and the 35th primer pair consisting of a base sequence represented by SEQ ID NO: 69 and 70, respectively;
p) at least one primer pair selected from the group consisting of the 36th primer pair consisting of a base sequence represented by SEQ ID NO: 71 and 72, respectively and the 37th primer pair consisting of a base sequence represented by SEQ ID NO: 73 and 74, respectively; and
q) at least one primer pair selected from the group consisting of the 38th primer pair consisting of a base sequence represented by SEQ ID NO: 75 and 76, respectively and the 39th primer pair consisting of a base sequence represented by SEQ ID NO: 77 and 78, respectively.

2. The primer set of claim 1 comprising:
the 1st primer pair consisting of a base sequence represented by SEQ ID NO: 1 and 2, respectively;
the 2nd primer pair consisting of a base sequence represented by SEQ ID NO: 3 and 4, respectively;
the 3rd primer pair consisting of a base sequence represented by SEQ ID NO: 5 and 6, respectively;
the 4th primer pair consisting of a base sequence represented by SEQ ID NO: 7 and 8, respectively;
the 5th primer pair consisting of a base sequence represented by SEQ ID NO: 9 and 10, respectively;
the 6th primer pair consisting of a base sequence represented by SEQ ID NO: 11 and 12, respectively;
the 7th primer pair consisting of a base sequence represented by SEQ ID NO: 13 and 14, respectively;
the 8th primer pair consisting of a base sequence represented by SEQ ID NO: 15 and 16, respectively;
the 9th primer pair consisting of a base sequence represented by SEQ ID NO: 17 and 18, respectively;
the 10th primer pair consisting of a base sequence represented by SEQ ID NO: 19 and 20, respectively;
the 11th primer pair consisting of a base sequence represented by SEQ ID NO: 21 and 22, respectively;
the 12th primer pair consisting of a base sequence represented by SEQ ID NO: 23 and 24, respectively;
the 13th primer pair consisting of a base sequence represented by SEQ ID NO: 25 and 26, respectively;
the 14th primer pair consisting of a base sequence represented by SEQ ID NO: 27 and 28, respectively;
the 15th primer pair consisting of a base sequence represented by SEQ ID NO: 29 and 30, respectively;
the 16th primer pair consisting of a base sequence represented by SEQ ID NO: 31 and 32, respectively;
the 17th primer pair consisting of a base sequence represented by SEQ ID NO: 33 and 34, respectively;
the 18th primer pair consisting of a base sequence represented by SEQ ID NO: 35 and 36, respectively;
the 19th primer pair consisting of a base sequence represented by SEQ ID NO: 37 and 38, respectively;
the 20th primer pair consisting of a base sequence represented by SEQ ID NO: 39 and 40, respectively;
the 21st primer pair consisting of a base sequence represented by SEQ ID NO: 41 and 42, respectively;
the 22nd primer pair consisting of a base sequence represented by SEQ ID NO: 43 and 44, respectively;
the 23rd primer pair consisting of a base sequence represented by SEQ ID NO: 45 and 46, respectively;
the 24th primer pair consisting of a base sequence represented by SEQ ID NO: 47 and 48, respectively;
the 25th primer pair consisting of a base sequence represented by SEQ ID NO: 49 and 50, respectively;
the 26th primer pair consisting of a base sequence represented by SEQ ID NO: 51 and 52, respectively;
the 27th primer pair consisting of a base sequence represented by SEQ ID NO: 53 and 54, respectively;
the 28th primer pair consisting of a base sequence represented by SEQ ID NO: 55 and 56, respectively;
the 29th primer pair consisting of a base sequence represented by SEQ ID NO: 57 and 58, respectively;
the 30th primer pair consisting of a base sequence represented by SEQ ID NO: 59 and 60, respectively;
the 31st primer pair consisting of a base sequence represented by SEQ ID NO: 61 and 62, respectively;
the 32nd primer pair consisting of a base sequence represented by SEQ ID NO: 63 and 64, respectively;
the 33rd primer pair consisting of a base sequence represented by SEQ ID NO: 65 and 66, respectively;
the 34th primer pair consisting of a base sequence represented by SEQ ID NO: 67 and 68, respectively;
the 35th primer pair consisting of a base sequence represented by SEQ ID NO: 69 and 70, respectively;
the 36th primer pair consisting of a base sequence represented by SEQ ID NO: 71 and 72, respectively;
the 37th primer pair consisting of a base sequence represented by SEQ ID NO: 73 and 74, respectively;
the 38th primer pair consisting of a base sequence represented by SEQ ID NO: 75 and 76, respectively; and
the 39th primer pair consisting of a base sequence represented by SEQ ID NO: 77 and 78, respectively.

3. The primer set of claim 1, wherein the detection is performed by next-generation sequencing (NGS).

4. The primer set of claim 1, wherein the food poisoning bacteria is at least one selected from the group consisting of *Bacillus, Campylobacter, Clostridium, Escherichia, Listeria, Salmonella, Staphylococcus, Vibrio,* and *Yersinia.*

5. The primer set of claim 1, wherein the food poisoning bacteria is at least one selected from the group consisting of *Bacillus cereus, Campylobacter coli, Campylobacter jejuni, Clostridium perfringens,* enteroaggregative *Escherichia coli* (EAEC), enterohemorrhagic *Escherichia coli* (EHEC), enteropathogenic *Escherichia coli* (EPEC), enteroinvasive *Escherichia coli* (EIEC), enterotoxigenic *Escherichia coli* (ETEC), *Listeria monocytogenes, Salmonella typhimurium, Staphylococcus aureus, Vibrio cholerae, Vibrio parahaemolyticus, Vibrio vulnificus* and *Yersinia enterocolitica.*

6. The primer set of claim 1 comprising:
i) at least one primer pair selected from the group consisting of the 1st primer pair consisting of a base sequence represented by SEQ ID NO: 1 and 2, respectively and the 2nd primer pair consisting of a base sequence represented by SEQ ID NO: 3 and 4, respectively, the at least one primer pair being capable of detecting *Bacillus cereus;*
ii) the 3rd primer pair consisting of a base sequence represented by SEQ ID NO: 5 and 6, respectively, the 3rd primer pair being capable of detecting *Campylobacter;*
iii) at least one primer pair selected from the group consisting of the 4th primer pair consisting of a base sequence represented by SEQ ID NO: 7 and 8, respectively and the 5th primer pair consisting of a base sequence represented by SEQ ID NO: 9 and 10, respectively, the at least one primer pair being capable of detecting *Campylobacter coli;*
iv) at least one primer pair selected from the group consisting of the 6th primer pair consisting of a base sequence represented by SEQ ID NO: 11 and 12, respectively and the 7th primer pair consisting of a base sequence represented by SEQ ID NO: 13 and 14, respectively, the at least one primer pair being capable of detecting *Campylobacter jejuni;*
v) at least one primer pair selected from the group consisting of the 8th primer pair consisting of a base sequence represented by SEQ ID NO: 15 and 16, respectively, the 9th primer pair consisting of a base sequence represented by SEQ ID NO: 17 and 18, respectively, and the 10th primer pair consisting of a base sequence represented by SEQ ID NO: 19 and 20, respectively, the at least one primer pair being capable of detecting Clostridium perfringens;
vi) the 11^{th} primer pair consisting of a base sequence represented by SEQ ID NO: 21 and 22, respectively, the 11^{th} primer pair being capable of detecting enteroaggregative *Escherichia coli* (EAEC);
vii) at least one primer pair selected from the group consisting of the 12th primer pair consisting of a base sequence represented by SEQ ID NO: 23 and 24, respectively, the 13th primer pair consisting of a base sequence represented by SEQ ID NO: 25 and 26, respectively, and the 14th primer pair consisting of a base sequence represented by SEQ ID NO: 27 and 28, respectively, the at least one primer pair being capable of detecting enterohemorrhagic *Escherichia coli* (EHEC);
viii) the 15th primer pair consisting of a base sequence represented by SEQ ID NO: 29 and 30, respectively, the 15th primer pair being capable of detecting enteropathogenic *Escherichia coli* (EPEC);
ix) at least one primer pair selected from the group consisting of the 16th primer pair consisting of a base sequence represented by SEQ ID NO: 31 and 32, respectively and the 17th primer pair consisting of a base sequence represented by SEQ ID NO: 33 and 34, respectively, the at least one primer pair being capable of detecting enteroinvasive *Escherichia coli* (EIEC);
x) the 18th primer pair consisting of a base sequence represented by SEQ ID NO: 35 and 36, respectively, the 18th primer pair being capable of detecting enterotoxigenic *Escherichia coli* (ETEC);
xi) at least one primer pair selected from the group consisting of the 19th primer pair consisting of a base sequence represented by SEQ ID NO: 37 and 38, respectively, the 20th primer pair consisting of a base sequence represented by SEQ ID NO: 39 and 40, respectively, and the 21st primer pair consisting of a base sequence represented by SEQ ID NO: 41 and 42, respectively, the at least one primer pair being capable of detecting *Listeria monocytogenes;*
xii) at least one primer pair selected from the group consisting of the 22nd primer pair consisting of a base sequence represented by SEQ ID NO: 43 and 44, respectively and the 23rd primer pair consisting of a base sequence represented by SEQ ID NO: 45 and 46, respectively, the at least one primer pair being capable of detecting *Salmonella typhimurium;*
xiii) at least one primer pair selected from the group consisting of the 24th primer pair consisting of a base sequence represented by SEQ ID NO: 47 and 48, respectively, the 25th primer pair consisting of a base sequence represented by SEQ ID NO: 49 and 50, respectively, the 26th primer pair consisting of a base sequence represented by SEQ ID NO: 51 and 52, respectively, the 27th primer pair consisting of a base sequence represented by SEQ ID NO: 53 and 54, respectively, and the 28th primer pair consisting of a base sequence represented by SEQ ID NO: 55 and 56, respectively, the at least one primer pair being capable of detecting *Staphylococcus aureus;*
xiv) at least one primer pair selected from the group consisting of the 29th primer pair consisting of a base sequence represented by SEQ ID NO: 57 and 58, respectively, the 30th primer pair consisting of a base sequence represented by SEQ ID NO: 59 and 60, respectively, and the 31st primer pair consisting of a base sequence represented by SEQ ID NO: 61 and 62, respectively, the at least one primer pair being capable of detecting *Vibrio cholerae;*
xv) at least one primer pair selected from the group consisting of the 32nd primer pair consisting of a base sequence represented by SEQ ID NO: 63 and 64, respectively, the 33rd primer pair consisting of a base sequence represented by SEQ ID NO: 65 and 66, respectively, the 34th primer pair consisting of a base sequence represented by SEQ ID NO: 67 and 68, respectively, and the 35th primer pair consisting of a base sequence represented by SEQ ID NO: 69 and 70, respectively, the at least one primer pair being capable of detecting *Vibrio parahaemolyticus;*
xvi) at least one primer pair selected from the group consisting of the 36th primer pair consisting of a base sequence represented by SEQ ID NO: 71 and 72, respectively and the 37th primer pair consisting of a base sequence represented by SEQ ID NO: 73 and 74, respectively, the at least one primer pair being capable of detecting *Vibrio vulnificus;* and
xvii) at least one primer pair selected from the group consisting of the 38th primer pair consisting of a base sequence represented by SEQ ID NO: 75 and 76, respectively and the 39th primer pair consisting of a base sequence represented by SEQ ID NO: 77 and 78, respectively, the at least one primer pair being capable of detecting *Yersinia enterocolitica.*

7. A composition for detecting food poisoning bacteria, the composition comprising the primer set of claim 1.

8. A next-generation sequencing panel for detecting food poisoning bacteria, the panel comprising the primer set of claim 1.

9. A kit for detecting food poisoning bacteria, the kit comprising the primer set of claim 1.

10. A next-generation sequencing method comprising the steps of: amplifying a target sequence by using the primer set of claim 1 and a sample;
reparing a library by using the amplified target sequence; and
performing a next-generation sequencing analysis by using the prepared library.

11. A method for detecting food poisoning bacteria, the method comprising the step of amplifying by using the primer set of claim 1 and a sample.

## Patentansprüche

1. Ein Primersatz zum Nachweisen von lebensmittelvergiftenden Bakterien, wobei der Primersatz Folgendes beinhaltet:
a) mindestens ein Primerpaar, ausgewählt aus der Gruppe, bestehend aus dem 1. Primerpaar, das aus einer durch SEQ ID NO: 1 bzw. 2 dargestellten Basensequenz besteht, und dem 2. Primerpaar, das aus einer durch SEQ ID NO: 3 bzw. 4 dargestellten Basensequenz besteht;
b) das 3. Primerpaar, das aus einer durch SEQ ID NO: 5 bzw. 6 dargestellten Basensequenz besteht;
c) mindestens ein Primerpaar, ausgewählt aus der Gruppe, bestehend aus dem 4. Primerpaar, das aus einer durch SEQ ID NO: 7 bzw. 8 dargestellten Basensequenz besteht, und dem 5. Primerpaar, das aus einer durch SEQ ID NO: 9 bzw. 10 dargestellten Basensequenz besteht;
d) mindestens ein Primerpaar, ausgewählt aus der Gruppe, bestehend aus dem 6. Primerpaar, das aus einer durch SEQ ID NO: 11 bzw. 12 dargestellten Basensequenz besteht, und dem 7. Primerpaar, das aus einer durch SEQ ID NO: 13 bzw. 14 dargestellten Basensequenz besteht;
e) mindestens ein Primerpaar, ausgewählt aus der Gruppe, bestehend aus dem 8. Primerpaar, das aus einer durch SEQ ID NO: 15 bzw. 16 dargestellten Basensequenz besteht, dem 9. Primerpaar, das aus einer durch SEQ ID NO: 17 bzw. 18 dargestellten Basensequenz besteht, und dem 10. Primerpaar, das aus einer durch SEQ ID NO: 19 bzw. 20 dargestellten Basensequenz besteht;
f) das 11. Primerpaar, das aus einer durch SEQ ID NO: 21 bzw. 22 dargestellten Basensequenz besteht;
g) mindestens ein Primerpaar, ausgewählt aus der Gruppe, bestehend aus dem 12. Primerpaar, das aus einer durch SEQ ID NO: 23 bzw. 24 dargestellten Basensequenz besteht, dem 13. Primerpaar, das aus einer durch SEQ ID NO: 25 bzw. 26 dargestellten Basensequenz besteht, und dem 14. Primerpaar, das aus einer durch SEQ ID NO: 27 bzw. 28 dargestellten Basensequenz besteht;
h) das 15. Primerpaar, das aus einer durch SEQ ID NO: 29 bzw. 30 dargestellten Basensequenz besteht;
i) mindestens ein Primerpaar, ausgewählt aus der Gruppe, bestehend aus dem 16. Primerpaar, das aus einer durch SEQ ID NO: 31 bzw. 32 dargestellten Basensequenz besteht, und dem 17. Primerpaar, das aus einer durch SEQ ID NO: 33 bzw. 34 dargestellten Basensequenz besteht;
j) das 18. Primerpaar, das aus einer durch SEQ ID NO: 35 bzw. 36 dargestellten Basensequenz besteht;
k) mindestens ein Primerpaar, ausgewählt aus der Gruppe, bestehend aus dem 19. Primerpaar, das aus einer durch SEQ ID NO: 37 bzw. 38 dargestellten Basensequenz besteht, dem 20. Primerpaar, das aus einer durch SEQ ID NO: 39 bzw. 40 dargestellten Basensequenz besteht, und dem 21. Primerpaar, das aus einer durch SEQ ID NO: 41 bzw. 42 dargestellten Basensequenz besteht;
l) mindestens ein Primerpaar, ausgewählt aus der Gruppe, bestehend aus dem 22. Primerpaar, das aus einer durch SEQ ID NO: 43 bzw. 44 dargestellten Basensequenz besteht, und dem 23. Primerpaar, das aus einer durch SEQ ID NO: 45 bzw. 46 dargestellten Basensequenz besteht;
m) mindestens ein Primerpaar, ausgewählt aus der Gruppe, bestehend aus dem 24. Primerpaar, das aus einer durch SEQ ID NO: 47 bzw. 48 dargestellten Basensequenz besteht, dem 25. Primerpaar, das aus einer durch SEQ ID NO: 49 bzw. 50 dargestellten Basensequenz besteht, dem 26. Primerpaar, das aus einer durch SEQ ID NO: 51 bzw. 52 dargestellten Basensequenz besteht, dem 27. Primerpaar, das aus einer durch SEQ ID NO: 53 bzw. 54 dargestellten Basensequenz besteht, und dem 28. Primerpaar, das aus einer durch SEQ ID NO: 55 bzw. 56 dargestellten Basensequenz besteht;
n) mindestens ein Primerpaar, ausgewählt aus der Gruppe, bestehend aus dem 29. Primerpaar, das aus einer durch SEQ ID NO: 57 bzw. 58 dargestellten Basensequenz besteht, dem 30. Primerpaar, das aus einer durch SEQ ID NO: 59 bzw. 60 dargestellten Basensequenz besteht, und dem 31. Primerpaar, das aus einer durch SEQ ID NO: 61 bzw. 62 dargestellten Basensequenz besteht;
o) mindestens ein Primerpaar, ausgewählt aus der Gruppe, bestehend aus dem 32. Primerpaar, das aus einer durch SEQ ID NO: 63 bzw. 64 dargestellten Basensequenz besteht, dem 33. Primerpaar, das aus einer durch SEQ ID NO: 65 bzw. 66 dargestellten Basensequenz besteht, dem 34. Primerpaar, das aus einer durch SEQ ID NO: 67 bzw. 68 dargestellten Basensequenz besteht, und dem 35. Primerpaar, das aus einer durch SEQ ID NO: 69 bzw. 70 dargestellten Basensequenz besteht;
p) mindestens ein Primerpaar, ausgewählt aus der Gruppe, bestehend aus dem 36. Primerpaar, das aus einer durch SEQ ID NO: 71 bzw. 72 dargestellten Basensequenz besteht, und dem 37. Primerpaar, das aus einer durch SEQ ID NO: 73 bzw. 74 dargestellten Basensequenz besteht; und
q) mindestens ein Primerpaar, ausgewählt aus der Gruppe, bestehend aus dem 38. Primerpaar, das aus einer durch SEQ ID NO: 75 bzw. 76 dargestellten Basensequenz besteht, und dem 39. Primerpaar, das aus einer durch SEQ ID NO: 77 bzw. 78 dargestellten Basensequenz besteht.

2. Primersatz gemäß Anspruch 1, der Folgendes beinhaltet:
das 1. Primerpaar, das aus einer durch SEQ ID NO: 1 bzw. 2 dargestellten Basensequenz besteht;
das 2. Primerpaar, das aus einer durch SEQ ID NO: 3 bzw. 4 dargestellten Basensequenz besteht;
das 3. Primerpaar, das aus einer durch SEQ ID NO: 5 bzw. 6 dargestellten Basensequenz besteht;
das 4. Primerpaar, das aus einer durch SEQ ID NO: 7 bzw. 8 dargestellten Basensequenz besteht;
das 5. Primerpaar, das aus einer durch SEQ ID NO: 9 bzw. 10 dargestellten Basensequenz besteht;
das 6. Primerpaar, das aus einer durch SEQ ID NO: 11 bzw. 12 dargestellten Basensequenz besteht;
das 7. Primerpaar, das aus einer durch SEQ ID NO: 13 bzw. 14 dargestellten Basensequenz besteht;
das 8. Primerpaar, das aus einer durch SEQ ID NO: 15 bzw. 16 dargestellten Basensequenz besteht;
das 9. Primerpaar, das aus einer durch SEQ ID NO: 17 bzw. 18 dargestellten Basensequenz besteht;
das 10. Primerpaar, das aus einer durch SEQ ID NO: 19 bzw. 20 dargestellten Basensequenz besteht;
das 11. Primerpaar, das aus einer durch SEQ ID NO: 21 bzw. 22 dargestellten Basensequenz besteht;
das 12. Primerpaar, das aus einer durch SEQ ID NO: 23 bzw. 24 dargestellten Basensequenz besteht;
das 13. Primerpaar, das aus einer durch SEQ ID NO: 25 bzw. 26 dargestellten Basensequenz besteht;
das 14. Primerpaar, das aus einer durch SEQ ID NO: 27 bzw. 28 dargestellten Basensequenz besteht;
das 15. Primerpaar, das aus einer durch SEQ ID NO: 29 bzw. 30 dargestellten Basensequenz besteht;
das 16. Primerpaar, das aus einer durch SEQ ID NO: 31 bzw. 32 dargestellten Basensequenz besteht;
das 17. Primerpaar, das aus einer durch SEQ ID NO: 33 bzw. 34 dargestellten Basensequenz besteht,
das 18. Primerpaar, das aus einer durch SEQ ID NO: 35 bzw. 36 dargestellten Basensequenz besteht;
das 19. Primerpaar, das aus einer durch SEQ ID NO: 37 bzw. 38 dargestellten Basensequenz besteht;
das 20. Primerpaar, das aus einer durch SEQ ID NO 39 bzw. 40 dargestellten Basensequenz besteht;
das 21. Primerpaar, das aus einer durch SEQ ID NO: 41 bzw. 42 dargestellten Basensequenz besteht;
das 22. Primerpaar, das aus einer durch SEQ ID NO: 43 bzw. 44 dargestellten Basensequenz besteht;
das 23. Primerpaar, das aus einer durch SEQ ID NO: 45 bzw. 46 dargestellten Basensequenz besteht;
das 24. Primerpaar, das aus einer durch SEQ ID NO: 47 bzw. 48 dargestellten Basensequenz besteht;
das 25. Primerpaar, das aus einer durch SEQ ID NO: 49 bzw. 50 dargestellten Basensequenz besteht;
das 26. Primerpaar, das aus einer durch SEQ ID NO: 51 bzw. 52 dargestellten Basensequenz besteht;
das 27. Primerpaar, das aus einer durch SEQ ID NO: 53 bzw. 54 dargestellten Basensequenz besteht;
das 28. Primerpaar, das aus einer durch SEQ ID NO: 55 bzw. 56 dargestellten Basensequenz besteht;
das 29. Primerpaar, das aus einer durch SEQ ID NO: 57 bzw. 58 dargestellten Basensequenz besteht;
das 30. Primerpaar, das aus einer durch SEQ ID NO: 59 bzw. 60 dargestellten Basensequenz besteht;
das 31. Primerpaar, das aus einer durch SEQ ID NO: 61 bzw. 62 dargestellten Basensequenz besteht;
das 32. Primerpaar, das aus einer durch SEQ ID NO: 63 bzw. 64 dargestellten Basensequenz besteht,
das 33. Primerpaar, das aus einer durch SEQ ID NO: 65 bzw. 66 dargestellten Basensequenz besteht;
das 34. Primerpaar, das aus einer durch SEQ ID NO: 67 bzw. 68 dargestellten Basensequenz besteht;
das 35. Primerpaar, das aus einer durch SEQ ID NO: 69 bzw. 70 dargestellten Basensequenz besteht;
das 36. Primerpaar, das aus einer durch SEQ ID NO: 71 bzw. 72 dargestellten Basensequenz besteht;
das 37. Primerpaar, das aus einer durch SEQ ID NO: 73 bzw. 74 dargestellten Basensequenz besteht;
das 38. Primerpaar, das aus einer durch SEQ ID NO: 75 bzw. 76 dargestellten Basensequenz besteht; und
das 39. Primerpaar, das aus einer durch SEQ ID NO: 77 bzw. 78 dargestellten Basensequenz besteht.

3. Primersatz gemäß Anspruch 1, wobei der Nachweis mittels Next-Generation-Sequenzierung (NGS) durchgeführt wird.

4. Primersatz gemäß Anspruch 1, wobei die lebensmittelvergiftenden Bakterien mindestens eine aus der Gruppe, bestehend aus *Bacillus, Campylobacter, Clostridium, Escherichia, Listeria, Salmonella, Staphylococcus, Vibrio* und *Yersinia,* ausgewählte sind.

5. Primersatz gemäß Anspruch 1, wobei die lebensmittelvergiftenden Bakterien mindestens eine aus der Gruppe, bestehend aus *Bacillus cereus, Campylobacter coli, Campylobacter jejuni, Clostridium perfringens,* enteroaggregativer *Escherichia coli* (EAEC), enterohämorrhagischer *Escherichia coli* (EHEC), enteropathogener *Escherichia coli* (EPEC), enteroinvasiver *Escherichia coli* (EIEC), enterotoxigener *Escherichia coli* (ETEC), *Listeria monocytogenes, Salmonella typhimurium, Staphylococcus aureus, Vibrio cholerae, Vibrio parahaemolyticus, Vibrio vulnificus* und *Yersinia enterocolitica,* ausgewählte sind.

6. Primersatz gemäß Anspruch 1, der Folgendes beinhaltet:
i) mindestens ein Primerpaar, ausgewählt aus der Gruppe, bestehend aus dem 1. Primerpaar, das aus einer durch SEQ ID NO: 1 bzw. 2 dargestellten Basensequenz besteht, und dem 2. Primerpaar, das aus einer durch SEQ ID NO: 3 bzw. 4 dargestellten Basensequenz besteht, wobei das mindestens eine Primerpaar in der Lage ist, *Bacillus cereus* nachzuweisen;
ii) das 3. Primerpaar, das aus einer durch SEQ ID NO: 5 bzw. 6 dargestellten Basensequenz besteht, wobei das 3. Primerpaar in der Lage ist, *Campylobacter* nachzuweisen;
iii) mindestens ein Primerpaar, ausgewählt aus der Gruppe, bestehend aus dem 4. Primerpaar, das aus einer durch SEQ ID NO: 7 bzw. 8 dargestellten Basensequenz besteht, und dem 5. Primerpaar, das aus einer durch SEQ ID NO: 9 bzw. 10 dargestellten Basensequenz besteht, wobei das mindestens eine Primerpaar in der Lage ist, *Campylobacter coli* nachzuweisen;
iv) mindestens ein Primerpaar, ausgewählt aus der Gruppe, bestehend aus dem 6. Primerpaar, das aus einer durch SEQ ID NO: 11 bzw. 12 dargestellten Basensequenz besteht, und dem 7. Primerpaar, das aus einer durch SEQ ID NO: 13 bzw. 14 dargestellten Basensequenz besteht, wobei das mindestens eine Primerpaar in der Lage ist, *Campylobacter jejuni* nachzuweisen;
v) mindestens ein Primerpaar, ausgewählt aus der Gruppe, bestehend aus dem 8. Primerpaar, das aus einer durch SEQ ID NO: 15 bzw. 16 dargestellten Basensequenz besteht, dem 9. Primerpaar, das aus einer durch SEQ ID NO: 17 bzw. 18 dargestellten Basensequenz besteht, und dem 10. Primerpaar, das aus einer durch SEQ ID NO: 19 bzw. 20 dargestellten Basensequenz besteht, wobei das mindestens eine Primerpaar in der Lage ist, Clostridium perfringens nachzuweisen;
vi) das 11. Primerpaar, das aus einer durch SEQ ID NO: 21 bzw. 22 dargestellten Basensequenz besteht, wobei das 11. Primerpaar in der Lage ist, enteroaggregative *Escherichia coli* (EAEC) nachzuweisen;
vii) mindestens ein Primerpaar, ausgewählt aus der Gruppe, bestehend aus dem 12. Primerpaar, das aus einer durch SEQ ID NO: 23 bzw. 24 dargestellten Basensequenz besteht, dem 13. Primerpaar, das aus einer durch SEQ ID NO: 25 bzw. 26 dargestellten Basensequenz besteht, und dem 14. Primerpaar, das aus einer durch SEQ ID NO: 27 bzw. 28 dargestellten Basensequenz besteht, wobei das mindestens eine Primerpaar in der Lage ist, enterohämorrhagische *Escherichia coli* (EHEC) nachzuweisen;
viii) das 15. Primerpaar, das aus einer durch SEQ ID NO: 29 bzw. 30 dargestellten Basensequenz besteht, wobei das 15. Primerpaar in der Lage ist, enteropathogene *Escherichia coli* (EPEC) nachzuweisen;
ix) mindestens ein Primerpaar, ausgewählt aus der Gruppe, bestehend aus dem 16. Primerpaar, das aus einer durch SEQ ID NO: 31 bzw. 32 dargestellten Basensequenz besteht, und dem 17. Primerpaar, das aus einer durch SEQ ID NO: 33 bzw. 34 dargestellten Basensequenz besteht, wobei das mindestens eine Primerpaar in der Lage ist, enteroinvasive *Escherichia coli* (EIEC) nachzuweisen;
x) das 18. Primerpaar, das aus einer durch SEQ ID NO: 35 bzw. 36 dargestellten Basensequenz besteht, wobei das 18. Primerpaar in der Lage ist, enterotoxigene *Escherichia coli* (ETEC) nachzuweisen;
xi) mindestens ein Primerpaar, ausgewählt aus der Gruppe, bestehend aus dem 19. Primerpaar, das aus einer durch SEQ ID NO: 37 bzw. 38 dargestellten Basensequenz besteht, dem 20. Primerpaar, das aus einer durch SEQ ID NO 39 bzw. 40 dargestellten Basensequenz besteht, und dem 21. Primerpaar, das aus einer durch SEQ ID NO: 41 bzw. 42 dargestellten Basensequenz besteht, wobei das mindestens eine Primerpaar in der Lage ist, *Listeria monocytogenes* nachzuweisen;
xii) mindestens ein Primerpaar, ausgewählt aus der Gruppe, bestehend aus dem 22. Primerpaar, das aus einer durch SEQ ID NO: 43 bzw. 44 dargestellten Basensequenz besteht, und dem 23. Primerpaar, das aus einer durch SEQ ID NO: 45 bzw. 46 dargestellten Basensequenz besteht, wobei das mindestens eine Primerpaar in der Lage ist, *Salmonella typhimurium* nachzuweisen;
xiii) mindestens ein Primerpaar, ausgewählt aus der Gruppe, bestehend aus dem 24. Primerpaar, das aus einer durch SEQ ID NO: 47 bzw. 48 dargestellten Basensequenz besteht, dem 25. Primerpaar, das aus einer durch SEQ ID NO: 49 bzw. 50 dargestellten Basensequenz besteht, dem 26. Primerpaar, das aus einer durch SEQ ID NO: 51 bzw. 52 dargestellten Basensequenz besteht, dem 27. Primerpaar, das aus einer durch SEQ ID NO: 53 bzw. 54 dargestellten Basensequenz besteht, und dem 28. Primerpaar, das aus einer durch SEQ ID NO: 55 bzw. 56 dargestellten Basensequenz besteht, wobei das mindestens eine Primerpaar in der Lage ist, *Staphylococcus aureus* nachzuweisen;
xiv) mindestens ein Primerpaar, ausgewählt aus der Gruppe, bestehend aus dem 29. Primerpaar, das aus einer durch SEQ ID NO: 57 bzw. 58 dargestellten Basensequenz besteht, dem 30. Primerpaar, das aus einer durch SEQ ID NO: 59 bzw. 60 dargestellten Basensequenz besteht, und dem 31. Primerpaar, das aus einer durch SEQ ID NO: 61 bzw. 62 dargestellten Basensequenz besteht, wobei das mindestens eine Primerpaar in der Lage ist, *Vibrio cholerae* nachzuweisen;
xv) mindestens ein Primerpaar, ausgewählt aus der Gruppe, bestehend aus dem 32. Primerpaar, das aus einer durch SEQ ID NO: 63 bzw. 64 dargestellten Basensequenz besteht, dem 33. Primerpaar, das aus einer durch SEQ ID NO: 65 bzw. 66 dargestellten Basensequenz besteht, dem 34. Primerpaar, das aus einer durch SEQ ID NO: 67 bzw. 68 dargestellten Basensequenz besteht, und dem 35. Primerpaar, das aus einer durch SEQ ID NO: 69 bzw. 70 dargestellten Basensequenz besteht, wobei das mindestens eine Primerpaar in der Lage ist, *Vibrio parahaemolyticus* nachzuweisen;
xvi) mindestens ein Primerpaar, ausgewählt aus der Gruppe, bestehend aus dem 36. Primerpaar, das aus einer durch SEQ ID NO: 71 bzw. 72 dargestellten Basensequenz besteht, und dem 37. Primerpaar, das aus einer durch SEQ ID NO: 73 bzw. 74 dargestellten Basensequenz besteht, wobei das mindestens eine Primerpaar in der Lage ist, *Vibrio vulnificus* nachzuweisen; und
xvii) mindestens ein Primerpaar, ausgewählt aus der Gruppe, bestehend aus dem 38. Primerpaar, das aus einer durch SEQ ID NO: 75 bzw. 76 dargestellten Basensequenz besteht, und dem 39. Primerpaar, das aus einer durch SEQ ID NO: 77 bzw. 78 dargestellten Basensequenz besteht, wobei das mindestens eine Primerpaar in der Lage ist, *Yersinia enterocolitica* nachzuweisen.

7. Eine Zusammensetzung zum Nachweisen von lebensmittelvergiftenden Bakterien, wobei die Zusammensetzung den Primersatz gemäß Anspruch 1 beinhaltet.

8. Ein Next-Generation-Sequenzierungspanel zum Nachweisen von lebensmittelvergiftenden Bakterien, wobei das Panel den Primersatz gemäß Anspruch 1 beinhaltet.

9. Ein Kit zum Nachweisen von lebensmittelvergiftenden Bakterien, wobei der Kit den Primersatz gemäß Anspruch 1 beinhaltet.

10. Ein Next-Generation-Sequenzierungsverfahren, das die folgenden Schritte beinhaltet:
Amplifizieren einer Zielsequenz durch Verwenden des Primersatzes gemäß Anspruch 1 und einer Probe;
Herstellen einer Bibliothek durch Verwenden der amplifizierten Zielsequenz; und
Durchführen einer Next-Generation-Sequenzierungsanalyse durch Verwenden der hergestellten Bibliothek.

11. Ein Verfahren zum Nachweisen von lebensmittelvergiftenden Bakterien, wobei das Verfahren den Schritt des Amplifizierens durch Verwenden des Primersatzes gemäß Anspruch 1 und einer Probe beinhaltet.

## Revendications

1. Un ensemble d'amorces pour détecter des bactéries d'intoxication alimentaire, l'ensemble d'amorces comprenant :
a) au moins une paire d'amorces sélectionnée dans le groupe constitué de la 1^{re} paire d'amorces constituées d'une séquence de bases représentée par SEQ ID NO : 1 et SEQ ID NO : 2, respectivement et de la 2^{e} paire d'amorces constituées d'une séquence de bases représentée par SEQ ID NO : 3 et SEQ ID NO : 4, respectivement ;
b) la 3^{e} paire d'amorces constituées d'une séquence de bases représentée par SEQ ID NO : 5 et SEQ ID NO : 6, respectivement ;
c) au moins une paire d'amorces sélectionnée dans le groupe constitué de la 4^{e} paire d'amorces constituées d'une séquence de bases représentée par SEQ ID NO : 7 et SEQ ID NO : 8, respectivement et de la 5^{e} paire d'amorces constituées d'une séquence de bases représentée par SEQ ID NO : 9 et SEQ ID NO : 10, respectivement ;
d) au moins une paire d'amorces sélectionnée dans le groupe constitué de la 6^{e} paire d'amorces constituées d'une séquence de bases représentée par SEQ ID NO : 11 et SEQ ID NO : 12, respectivement et de la 7^{e} paire d'amorces constituées d'une séquence de bases représentée par SEQ ID NO : 13 et SEQ ID NO : 14, respectivement ;
e) au moins une paire d'amorces sélectionnée dans le groupe constitué de la 8^{e} paire d'amorces constituées d'une séquence de bases représentée par SEQ ID NO : 15 et SEQ ID NO : 16, respectivement, de la 9^{e} paire d'amorces constituées d'une séquence de bases représentée par SEQ ID NO : 17 et SEQ ID NO : 18, respectivement, et de la 10^{e} paire d'amorces constituées d'une séquence de bases représentée par SEQ ID NO : 19 et SEQ ID NO : 20, respectivement ;
f) la 11^{e} paire d'amorces constituées d'une séquence de bases représentée par SEQ ID NO : 21 et SEQ ID NO : 22, respectivement ;
g) au moins une paire d'amorces sélectionnée dans le groupe constitué de la 12^{e} paire d'amorces constituées d'une séquence de bases représentée par SEQ ID NO : 23 et SEQ ID NO : 24, respectivement, de la 13^{e} paire d'amorces constituées d'une séquence de bases représentée par SEQ ID NO : 25 et SEQ ID NO : 26, respectivement, et de la 14^{e} paire d'amorces constituées d'une séquence de bases représentée par SEQ ID NO : 27 et SEQ ID NO : 28, respectivement ;
h) la 15^{e} paire d'amorces constituées d'une séquence de bases représentée par SEQ ID NO : 29 et SEQ ID NO : 30, respectivement ;
i) au moins une paire d'amorces sélectionnée dans le groupe constitué de la 16^{e} paire d'amorces constituées d'une séquence de bases représentée par SEQ ID NO : 31 et SEQ ID NO : 32, respectivement et de la 17^{e} paire d'amorces constituées d'une séquence de bases représentée par SEQ ID NO : 33 et SEQ ID NO : 34, respectivement ;
j) la 18^{e} paire d'amorces constituées d'une séquence de bases représentée par SEQ ID NO : 35 et SEQ ID NO : 36, respectivement ;
k) au moins une paire d'amorces sélectionnée dans le groupe constitué de la 19^{e} paire d'amorces constituées d'une séquence de bases représentée par SEQ ID NO : 37 et SEQ ID NO : 38, respectivement, de la 20^{e} paire d'amorces constituées d'une séquence de bases représentée par SEQ ID NO : 39 et SEQ ID NO : 40, respectivement, et de la 21^{e} paire d'amorces constituées d'une séquence de bases représentée par SEQ ID NO : 41 et SEQ ID NO : 42, respectivement ;
l) au moins une paire d'amorces sélectionnée dans le groupe constitué de la 22^{e} paire d'amorces constituées d'une séquence de bases représentée par SEQ ID NO : 43 et SEQ ID NO : 44, respectivement et de la 23^{e} paire d'amorces constituées d'une séquence de bases représentée par SEQ ID NO : 45 et SEQ ID NO : 46, respectivement ;
m) au moins une paire d'amorces sélectionnée dans le groupe constitué de la 24^{e} paire d'amorces constituées d'une séquence de bases représentée par SEQ ID NO : 47 et SEQ ID NO : 48, respectivement, de la 25^{e} paire d'amorces constituées d'une séquence de bases représentée par SEQ ID NO : 49 et SEQ ID NO : 50, respectivement, de la 26^{e} paire d'amorces constituées d'une séquence de bases représentée par SEQ ID NO : 51 et SEQ ID NO : 52, respectivement, de la 27^{e} paire d'amorces constituées d'une séquence de bases représentée par SEQ ID NO : 53 et SEQ ID NO : 54, respectivement, et de la 28^{e} paire d'amorces constituées d'une séquence de bases représentée par SEQ ID NO : 55 et SEQ ID NO : 56, respectivement ;
n) au moins une paire d'amorces sélectionnée dans le groupe constitué de la 29^{e} paire d'amorces constituées d'une séquence de bases représentée par SEQ ID NO : 57 et SEQ ID NO : 58, respectivement, de la 30^{e} paire d'amorces constituées d'une séquence de bases représentée par SEQ ID NO : 59 et SEQ ID NO : 60, respectivement, et de la 31^{e} paire d'amorces constituées d'une séquence de bases représentée par SEQ ID NO : 61 et SEQ ID NO : 62, respectivement ;
o) au moins une paire d'amorces sélectionnée dans le groupe constitué de la 32^{e} paire d'amorces constituées d'une séquence de bases représentée par SEQ ID NO : 63 et SEQ ID NO : 64, respectivement, de la 33^{e} paire d'amorces constituées d'une séquence de bases représentée par SEQ ID NO : 65 et SEQ ID NO : 66, respectivement, de la 34^{e} paire d'amorces constituées d'une séquence de bases représentée par SEQ ID NO : 67 et SEQ ID NO : 68, respectivement, et de la 35^{e} paire d'amorces constituées d'une séquence de bases représentée par SEQ ID NO : 69 et SEQ ID NO : 70, respectivement ;
p) au moins une paire d'amorces sélectionnée dans le groupe constitué de la 36^{e} paire d'amorces constituées d'une séquence de bases représentée par SEQ ID NO : 71 et SEQ ID NO : 72, respectivement et de la 37^{e} paire d'amorces constituées d'une séquence de bases représentée par SEQ ID NO : 73 et SEQ ID NO : 74, respectivement ; et
q) au moins une paire d'amorces sélectionnée dans le groupe constitué de la 38^{e} paire d'amorces constituées d'une séquence de bases représentée par SEQ ID NO : 75 et SEQ ID NO : 76, respectivement et de la 39^{e} paire d'amorces constituées d'une séquence de bases représentée par SEQ ID NO : 77 et SEQ ID NO : 78, respectivement.

2. L'ensemble d'amorces de la revendication 1 comprenant :
la 1^{re} paire d'amorces constituées d'une séquence de bases représentée par SEQ ID NO : 1 et SEQ ID NO : 2, respectivement ;
la 2^{e} paire d'amorces constituées d'une séquence de bases représentée par SEQ ID NO : 3 et SEQ ID NO : 4, respectivement ;
la 3^{e} paire d'amorces constituées d'une séquence de bases représentée par SEQ ID NO : 5 et SEQ ID NO : 6, respectivement ;
la 4^{e} paire d'amorces constituées d'une séquence de bases représentée par SEQ ID NO : 7 et SEQ ID NO : 8, respectivement ;
la 5^{e} paire d'amorces constituées d'une séquence de bases représentée par SEQ ID NO : 9 et SEQ ID NO : 10, respectivement ;
la 6^{e} paire d'amorces constituées d'une séquence de bases représentée par SEQ ID NO : 11 et SEQ ID NO : 12, respectivement ;
la 7^{e} paire d'amorces constituées d'une séquence de bases représentée par SEQ ID NO : 13 et SEQ ID NO : 14, respectivement ;
la 8^{e} paire d'amorces constituées d'une séquence de bases représentée par SEQ ID NO : 15 et SEQ ID NO : 16, respectivement ;
la 9^{e} paire d'amorces constituées d'une séquence de bases représentée par SEQ ID NO : 17 et SEQ ID NO : 18, respectivement ;
la 10^{e} paire d'amorces constituées d'une séquence de bases représentée par SEQ ID NO : 19 et SEQ ID NO : 20, respectivement ;
la 11^{e} paire d'amorces constituées d'une séquence de bases représentée par SEQ ID NO : 21 et SEQ ID NO : 22, respectivement ;
la 12^{e} paire d'amorces constituées d'une séquence de bases représentée par SEQ ID NO : 23 et SEQ ID NO : 24, respectivement ;
la 13^{e} paire d'amorces constituées d'une séquence de bases représentée par SEQ ID NO : 25 et SEQ ID NO : 26, respectivement ;
la 14^{e} paire d'amorces constituées d'une séquence de bases représentée par SEQ ID NO : 27 et SEQ ID NO : 28, respectivement ;
la 15^{e} paire d'amorces constituées d'une séquence de bases représentée par SEQ ID NO : 29 et SEQ ID NO : 30, respectivement ;
la 16^{e} paire d'amorces constituées d'une séquence de bases représentée par SEQ ID NO : 31 et SEQ ID NO : 32, respectivement ;
la 17^{e} paire d'amorces constituées d'une séquence de bases représentée par SEQ ID NO : 33 et SEQ ID NO : 34, respectivement ;
la 18^{e} paire d'amorces constituées d'une séquence de bases représentée par SEQ ID NO : 35 et SEQ ID NO : 36, respectivement ;
la 19^{e} paire d'amorces constituées d'une séquence de bases représentée par SEQ ID NO : 37 et SEQ ID NO : 38, respectivement ;
la 20^{e} paire d'amorces constituées d'une séquence de bases représentée par SEQ ID NO : 39 et SEQ ID NO : 40, respectivement ;
la 21^{e} paire d'amorces constituées d'une séquence de bases représentée par SEQ ID NO : 41 et SEQ ID NO : 42, respectivement ;
la 22^{e} paire d'amorces constituées d'une séquence de bases représentée par SEQ ID NO : 43 et SEQ ID NO : 44, respectivement ;
la 23^{e} paire d'amorces constituées d'une séquence de bases représentée par SEQ ID NO : 45 et SEQ ID NO : 46, respectivement ;
la 24^{e} paire d'amorces constituées d'une séquence de bases représentée par SEQ ID NO : 47 et SEQ ID NO : 48, respectivement ;
la 25^{e} paire d'amorces constituées d'une séquence de bases représentée par SEQ ID NO : 49 et SEQ ID NO : 50, respectivement ;
la 26^{e} paire d'amorces constituées d'une séquence de bases représentée par SEQ ID NO : 51 et SEQ ID NO : 52, respectivement ;
la 27^{e} paire d'amorces constituées d'une séquence de bases représentée par SEQ ID NO : 53 et SEQ ID NO : 54, respectivement ;
la 28^{e} paire d'amorces constituées d'une séquence de bases représentée par SEQ ID NO : 55 et SEQ ID NO : 56, respectivement ;
la 29^{e} paire d'amorces constituées d'une séquence de bases représentée par SEQ ID NO : 57 et SEQ ID NO : 58, respectivement ;
la 30^{e} paire d'amorces constituées d'une séquence de bases représentée par SEQ ID NO : 59 et SEQ ID NO : 60, respectivement ;
la 31^{e} paire d'amorces constituées d'une séquence de bases représentée par SEQ ID NO : 61 et SEQ ID NO : 62, respectivement ;
la 32^{e} paire d'amorces constituées d'une séquence de bases représentée par SEQ ID NO : 63 et SEQ ID NO : 64, respectivement ;
la 33^{e} paire d'amorces constituées d'une séquence de bases représentée par SEQ ID NO : 65 et SEQ ID NO : 66, respectivement ;
la 34^{e} paire d'amorces constituées d'une séquence de bases représentée par SEQ ID NO : 67 et SEQ ID NO : 68, respectivement ;
la 35^{e} paire d'amorces constituées d'une séquence de bases représentée par SEQ ID NO : 69 et SEQ ID NO : 70, respectivement ;
la 36^{e} paire d'amorces constituées d'une séquence de bases représentée par SEQ ID NO : 71 et SEQ ID NO : 72, respectivement ;
la 37^{e} paire d'amorces constituées d'une séquence de bases représentée par SEQ ID NO : 73 et SEQ ID NO : 74, respectivement ;
la 38^{e} paire d'amorces constituées d'une séquence de bases représentée par SEQ ID NO : 75 et SEQ ID NO : 76, respectivement ; et
la 39^{e} paire d'amorces constituées d'une séquence de bases représentée par SEQ ID NO : 77 et SEQ ID NO : 78, respectivement.

3. L'ensemble d'amorces de la revendication 1, dans lequel la détection est réalisée par séquençage de nouvelle génération (SNG).

4. L'ensemble d'amorces de la revendication 1, dans lequel les bactéries d'intoxication alimentaire sont d'au moins un genre sélectionné dans le groupe constitué de *Bacillus,* de *Campylobacter,* de *Clostridium, d'Escherichia,* de *Listeria,* de *Salmonella,* de *Staphylococcus,* de *Vibrio,* et de *Yersinia.*

5. L'ensemble d'amorces de la revendication 1, dans lequel les bactéries d'intoxication alimentaire sont d'au moins une souche sélectionnée dans le groupe constitué de *Bacillus cereus,* de *Campylobacter coli,* de *Campylobacter jejuni,* de *Clostridium perfringens, d'Escherichia coli* entéroagrégatif (ECEA), *d'Escherichia coli* entérohémorragique (ECEH), *d'Escherichia coli* entéropathogène (ECEP), *d'Escherichia coli* entéroinvasif (ECEI), *d'Escherichia coli* entérotoxigène (ECET), de *Listeria monocytogenes,* de *Salmonella typhimurium,* de *Staphylococcus aureus,* de *Vibrio cholerae,* de *Vibrio parahaemolyticus,* de *Vibrio vulnificus* et de *Yersinia enterocolitica.*

6. L'ensemble d'amorces de la revendication 1 comprenant :
i) au moins une paire d'amorces sélectionnée dans le groupe constitué de la 1^{re} paire d'amorces constituées d'une séquence de bases représentée par SEQ ID NO : 1 et SEQ ID NO : 2, respectivement et de la 2^{e} paire d'amorces constituées d'une séquence de bases représentée par SEQ ID NO : 3 et SEQ ID NO : 4, respectivement, l'au moins une paire d'amorces étant capable de détecter *Bacillus cereus ;*
ii) la 3^{e} paire d'amorces constituées d'une séquence de bases représentée par SEQ ID NO : 5 et SEQ ID NO : 6, respectivement, la 3^{e} paire d'amorces étant capable de détecter *Campylobacter;*
iii) au moins une paire d'amorces sélectionnée dans le groupe constitué de la 4^{e} paire d'amorces constituées d'une séquence de bases représentée par SEQ ID NO : 7 et SEQ ID NO : 8, respectivement et de la 5^{e} paire d'amorces constituées d'une séquence de bases représentée par SEQ ID NO : 9 et SEQ ID NO : 10, respectivement, l'au moins une paire d'amorces étant capable de détecter *Campylobacter coli ;*
iv) au moins une paire d'amorces sélectionnée dans le groupe constitué de la 6^{e} paire d'amorces constituées d'une séquence de bases représentée par SEQ ID NO : 11 et SEQ ID NO : 12, respectivement et de la 7^{e} paire d'amorces constituées d'une séquence de bases représentée par SEQ ID NO : 13 et SEQ ID NO : 14, respectivement, l'au moins une paire d'amorces étant capable de détecter *Campylobacter jejuni ;*
v) au moins une paire d'amorces sélectionnée dans le groupe constitué de la 8^{e} paire d'amorces constituées d'une séquence de bases représentée par SEQ ID NO : 15 et SEQ ID NO : 16, respectivement, de la 9^{e} paire d'amorces constituées d'une séquence de bases représentée par SEQ ID NO : 17 et SEQ ID NO : 18, respectivement, et de la 10^{e} paire d'amorces constituées d'une séquence de bases représentée par SEQ ID NO : 19 et SEQ ID NO : 20, respectivement, l'au moins une paire d'amorces étant capable de détecter *Clostridium perfringens ;*
vi) la 11^{e} paire d'amorces constituées d'une séquence de bases représentée par SEQ ID NO : 21 et SEQ ID NO : 22, respectivement, la 11^{e} paire d'amorces étant capable de détecter *Escherichia coli* entéroagrégatif (ECEA) ;
vii) au moins une paire d'amorces sélectionnée dans le groupe constitué de la 12^{e} paire d'amorces constituées d'une séquence de bases représentée par SEQ ID NO : 23 et SEQ ID NO : 24, respectivement, de la 13^{e} paire d'amorces constituées d'une séquence de bases représentée par SEQ ID NO : 25 et SEQ ID NO : 26, respectivement, et de la 14^{e} paire d'amorces constituées d'une séquence de bases représentée par SEQ ID NO : 27 et SEQ ID NO : 28, respectivement, l'au moins une paire d'amorces étant capable de détecter *Escherichia coli* entérohémorragique (ECEH) ;
viii) la 15^{e} paire d'amorces constituées d'une séquence de bases représentée par SEQ ID NO : 29 et SEQ ID NO : 30, respectivement, la 15^{e} paire d'amorces étant capable de détecter *Escherichia coli* entéropathogène (ECEP) ;
ix) au moins une paire d'amorces sélectionnée dans le groupe constitué de la 16^{e} paire d'amorces constituées d'une séquence de bases représentée par SEQ ID NO : 31 et SEQ ID NO : 32, respectivement et de la 17^{e} paire d'amorces constituées d'une séquence de bases représentée par SEQ ID NO : 33 et SEQ ID NO : 34, respectivement, l'au moins une paire d'amorces étant capable de détecter *Escherichia coli* entéroinvasif (ECEI) ;
x) la 18^{e} paire d'amorces constituées d'une séquence de bases représentée par SEQ ID NO : 35 et SEQ ID NO : 36, respectivement, la 18^{e} paire d'amorces étant capable de détecter *Escherichia coli* entérotoxigène (ECET) ;
xi) au moins une paire d'amorces sélectionnée dans le groupe constitué de la 19^{e} paire d'amorces constituées d'une séquence de bases représentée par SEQ ID NO : 37 et SEQ ID NO : 38, respectivement, de la 20^{e} paire d'amorces constituées d'une séquence de bases représentée par SEQ ID NO : 39 et SEQ ID NO : 40, respectivement, et de la 21^{e} paire d'amorces constituées d'une séquence de bases représentée par SEQ ID NO : 41 et SEQ ID NO : 42, respectivement, l'au moins une paire d'amorces étant capable de détecter *Listeria monocytogenes ;*
xii) au moins une paire d'amorces sélectionnée dans le groupe constitué de la 22^{e} paire d'amorces constituées d'une séquence de bases représentée par SEQ ID NO : 43 et SEQ ID NO : 44, respectivement et de la 23^{e} paire d'amorces constituées d'une séquence de bases représentée par SEQ ID NO : 45 et SEQ ID NO : 46, respectivement, l'au moins une paire d'amorces étant capable de détecter *Salmonella typhimurium ;*
xiii) au moins une paire d'amorces sélectionnée dans le groupe constitué de la 24^{e} paire d'amorces constituées d'une séquence de bases représentée par SEQ ID NO : 47 et SEQ ID NO : 48, respectivement, de la 25^{e} paire d'amorces constituées d'une séquence de bases représentée par SEQ ID NO : 49 et SEQ ID NO : 50, respectivement, de la 26^{e} paire d'amorces constituées d'une séquence de bases représentée par SEQ ID NO : 51 et SEQ ID NO : 52, respectivement, de la 27^{e} paire d'amorces constituées d'une séquence de bases représentée par SEQ ID NO : 53 et SEQ ID NO : 54, respectivement, et de la 28^{e} paire d'amorces constituées d'une séquence de bases représentée par SEQ ID NO : 55 et SEQ ID NO : 56, respectivement, l'au moins une paire d'amorces étant capable de détecter *Staphylococcus aureus ;*
xiv) au moins une paire d'amorces sélectionnée dans le groupe constitué de la 29^{e} paire d'amorces constituées d'une séquence de bases représentée par SEQ ID NO : 57 et SEQ ID NO : 58, respectivement, de la 30^{e} paire d'amorces constituées d'une séquence de bases représentée par SEQ ID NO : 59 et SEQ ID NO : 60, respectivement, et de la 31^{e} paire d'amorces constituées d'une séquence de bases représentée par SEQ ID NO : 61 et SEQ ID NO : 62, respectivement, l'au moins une paire d'amorces étant capable de détecter *Vibrio cholerae* ;
xv) au moins une paire d'amorces sélectionnée dans le groupe constitué de la 32^{e} paire d'amorces constituées d'une séquence de bases représentée par SEQ ID NO : 63 et SEQ ID NO : 64, respectivement, de la 33^{e} paire d'amorces constituées d'une séquence de bases représentée par SEQ ID NO : 65 et SEQ ID NO : 66, respectivement, de la 34^{e} paire d'amorces constituées d'une séquence de bases représentée par SEQ ID NO : 67 et SEQ ID NO : 68, respectivement, et de la 35^{e} paire d'amorces constituées d'une séquence de bases représentée par SEQ ID NO : 69 et SEQ ID NO : 70, respectivement, l'au moins une paire d'amorces étant capable de détecter *Vibrio parahaemolyticus* ;
xvi) au moins une paire d'amorces sélectionnée dans le groupe constitué de la 36^{e} paire d'amorces constituées d'une séquence de bases représentée par SEQ ID NO : 71 et SEQ ID NO : 72, respectivement et de la 37^{e} paire d'amorces constituées d'une séquence de bases représentée par SEQ ID NO : 73 et SEQ ID NO : 74, respectivement, l'au moins une paire d'amorces étant capable de détecter *Vibrio vulnificus* ; et
xvii) au moins une paire d'amorces sélectionnée dans le groupe constitué de la 38^{e} paire d'amorces constituées d'une séquence de bases représentée par SEQ ID NO : 75 et SEQ ID NO : 76, respectivement et de la 39^{e} paire d'amorces constituées d'une séquence de bases représentée par SEQ ID NO : 77 et SEQ ID NO : 78, respectivement, l'au moins une paire d'amorces étant capable de détecter *Yersinia enterocolitica.*

7. Une composition pour détecter des bactéries d'intoxication alimentaire, la composition comprenant l'ensemble d'amorces de la revendication 1.

8. Un panel de séquençage de nouvelle génération pour détecter des bactéries d'intoxication alimentaire, le panel comprenant l'ensemble d'amorces de la revendication 1.

9. Une trousse pour détecter des bactéries d'intoxication alimentaire, la trousse comprenant l'ensemble d'amorces de la revendication 1.

10. Un procédé de séquençage de nouvelle génération comprenant les étapes :
d'amplification d'une séquence cible à l'aide de l'ensemble d'amorces de la revendication 1 et d'un échantillon ;
de préparation d'une banque à l'aide de la séquence cible amplifiée ; et
de réalisation d'une analyse par séquençage de nouvelle génération à l'aide de la banque préparée.

11. Un procédé pour détecter des bactéries d'intoxication alimentaire, le procédé comprenant l'étape d'amplification à l'aide de l'ensemble d'amorces de la revendication 1 et d'un échantillon.
